(19) Europäisches Patentamt
European Patent Office
Office européen des brevets

(11) EP 3 121 282 B1

(12) # EUROPEAN PATENT SPECIFICATION

(45) Date of publication and mention of the grant of the patent:
**06.11.2019 Bulletin 2019/45**

(21) Application number: **15765171.2**

(22) Date of filing: **16.03.2015**

(51) Int Cl.:
*A61K 38/00* (2006.01) *A61K 38/19* (2006.01)
*A61P 25/18* (2006.01) *A01K 67/027* (2006.01)
*A61K 48/00* (2006.01) *C07K 14/52* (2006.01)

(86) International application number:
**PCT/KR2015/002527**

(87) International publication number:
**WO 2015/142012 (24.09.2015 Gazette 2015/38)**

(54) **NOVEL SAMDORI-2 GENE AND USE THEREOF**

NEUARTIGES SAMDORI-2-GEN UND VERWENDUNG DAVON

NOUVEAU GÈNE SAMDORI-2 ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2014 KR 20140031448**

(43) Date of publication of application:
**25.01.2017 Bulletin 2017/04**

(73) Proprietor: **NeuroMind Co., Ltd.**
**Yuseong-gu**
**Daejeon 34107 (KR)**

(72) Inventors:
- **KIM, Cheol-Hee**
  **Daejeon 305-755 (KR)**
- **PARK, Doo-Sang**
  **Daejeon 305-806 (KR)**
- **CHOI, Jung-Hwa**
  **Daejeon 302-739 (KR)**

(74) Representative: **EP&C**
**P.O. Box 3241**
**2280 GE Rijswijk (NL)**

(56) References cited:
**KR-A- 20130 094 255**

- **BORG K ET AL: "Molecular analysis of a constitutional complex genome rearrangement with 11 breakpoints involving chromosomes 3, 11, 12, and 21 and a 0.5-Mb submicroscopic deletion in a patient with mild mental retardation", HUMAN GENETICS 200511 DE, vol. 118, no. 2, November 2005 (2005-11), pages 267-275, XP002778042, ISSN: 0340-6717**
- **TOM TANG Y ET AL: "TAFA: a novel secreted family with conserved cysteine residues and restricted expression in the brain", GENO, ACADEMIC PRESS, SAN DIEGO, US, vol. 83, no. 4, 1 April 2004 (2004-04-01), pages 727-734, XP004496003, ISSN: 0888-7543, DOI: 10.1016/J.YGENO.2003.10.006**
- **KIM L ET AL: "Anxiety, hyperactivity and stereotypy in a zebrafish model of fragile X syndrome and autism spectrum disorder", PROGRESS IN NEURO-PSYCHOPHARMACOLOGY AND BIOLOGICAL PSYCHIATRY 20141203 ELSEVIER INC. USA, vol. 55, 3 December 2014 (2014-12-03), pages 40-49, XP002778043, ISSN: 0278-5846**
- **DATABASE GENBANK [Online] 11 January 2014 'Homo sapiens family with sequence similarity 19 (chemokine (C-C motif)-like), member A1 (FAM19A1), transcript variant 1, mRNA', XP055225731 Database accession no. NM_213609**
- **DATABASE GENBANK [Online] 11 January 2014 'Homo sapiens family with sequence similarity 19 (chemokine (C-C motif)-like), member A2 (FAM19A2), mRNA', XP055225735 Database accession no. NM_178539**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE GENBANK [Online] 11 January 2014 'Homo sapiens family with sequence similarity 19 (chemokine (C-C motif)-like), member A3 (FAM19A3), transcript variant 1, mRNA', XP055225739 Database accession no. NM_182759**

<110> Foundation of Research & Business Korea Research Institute of Bioscience and Biotechnology

<120> Novel Samdori2 gene and using thereof

<130> 2015FP0-02-007/PCT

<150> KR 2014/0031448
<151> 2014-03-18

<160> 28

<170> KopatentIn 2.0

<210> 1
<211> 1937
<212> DNA
<213> sam1_cDNA

<400> 1

```
cccgaatgca ctggagtggg gatggtccat cggcaactat aaactgattc tcatcaggaa      60
actgcacatt atctccccat cacttcaaag gtctcgtcag gcagaggtga cgccaggaga     120
tgatttaaag gtgaaaatga caaggtttcc acccctcaaa ccttggctcc ttttctgaca     180
atacagtctg aatgaacccg atgtcttttt ttttactgtg gaaataggat cggaagagag     240
taacattttt ttttttttaa tcctgataaa gaagattgtt gggaagctct ttgaaaaaaa     300
atttaaatt gtggcacaga tggattttaa aaagtgttag atctttccaa tgaacactaa      360
tagagtactc tgctcttggc tggatttttc agagaatggc aatggtctct gcgatgtcct     420
gggtcctgta tttgtggata agtgcttgtg caatgctact ctgccatgga tcccttcagc     480
acactttcca gcagcatcac ctgcacagac cagaaggagg gacgtgtgaa gtgatagcag     540
cacaccgatg ttgtaacaag aatcgcattg aggagcggtc acaaacagta aagtgttcct     600
gtctacctgg aaaagtggct ggaacaacaa gaaaccggcc ttcttgcgtc gatgcctcca     660
tagtgattgg gaaatggtgg tgtgagatgg agccttgcct agaaggagaa gaatgtaaga     720
cactccctga caattctgga tggatgtgcg caacaggcaa caaaattaag accacgagaa     780
ttcacccaag aacctaacag aagcatttgt ggtagtaaag gaaaaccaac cctctggaaa     840
atacattttg agaatctcaa acatctcaca tatatacaag ccaaatggat ttcttacttg     900
cactttgact ggctaccaga taatcacagt gcgtttactg tgtgtaacga aatatcctac     960
agtgagaaga cacagcgttt tggcaacacc atggaaagtg ggcttaaaaa agggttttct    1020
cagtgaaatt tttgggcatc atgaagaacg atcaactatc ttctaatttg aatctatagt    1080
tactttgtac catttgaaat atatgtatat atatatatat aatattttga aatattatct    1140
attctcttca agaaatgaac agtaccacag tttgagacgg ctggtgtacc cctttgagtt    1200
ttggatgttt tgtctgtttt gctttgtttt gttagtcatt tctttttcta acggcaagga    1260
agatatgtgc ccttttgaga attcaagatg gcactgacac gggaaggcca gctacaggtg    1320
gactcctgga atttgaggca tcataatgat actgaatcaa gaacttcctt ctgcttctac    1380
cagatggccc aaggaagcac atcgtcctgt tttattgctt tctaccctgt gcaatattag    1440
```

catgcaagct tggcttacat agtcatactt tatattcaat tgatatataa taaccgttct 1500
aacctcttcc aggaaaatat ttttagaact actagctttt ccacttagaa gaaaatgagg 1560
attcttaagg gagccactcc accatgctat taagactctg gcagagttat gggtaggata 1620
tggatcccta catgaataag tcctgtaaat acaatgtctt aaggctttgt atagctgtcc 1680
tagactgcag aaatgtcctc tgattaaatc caaagtctgg catcgttaac tacatagtgc 1740
tgtagcaaca agtcttatca tggcatctct ttctatgttt ggtttgcttt ttccaagagt 1800
attcaggtct cctcttgtga gataggaagg ccatgaaaac aattagattt caagatgatc 1860
tatgtgacca aatgttggac agccctatta aagtggtaaa caacttcttt ctaaaaaaaa 1920
aaaaaaaaaa aaaaaaa 1937

<210> 2
<211> 4069
<212> DNA
<213> sam2_cDNA

<400> 2

```
gcagttgctg gggctaacag aactggctgt tgggagagag aggcgaggca acgccgcccg      60
gccctgccat tccattttac tgcttaactc aacctggagt gtcaggacct attctcctgc     120
attctcgctg cactatacgg gggagccaag tcactctgta ctcactggac agccaggctg     180
ggtggaatag ggttctagag tggagcaggg gctctgccct ttatggcgac tttctaaaag     240
attagcatca gtgtcttttg acagtatagc atctggggtg ggacgcggag aggaggcttt     300
gcttgctaaa cggcttcttt ctccgccggt gatctggatt tgtttcctcg gccccctccc     360
cccgctcctt tctttctccc ctcccgcctt tagcaaagtg acacaggcga cacctgctcg     420
cttgtgttcg atgttggaac tcgcacctcc tcggtggtga cggtgccagg gcactgctac     480
caggggggaca tcggcgggtt tcccttcctt ccagcccacg atgcctagaa gggcagcggg    540
tgggctgcgt ggggctctgc ccgcagcact tcgaggcggg attgaggggc tgaagctggc     600
caggagttgc tgctaagtgg attcgagtgg aaggcgccaa gtctccgcga gggcaccagc     660
ggggacctat ctgcgagcag tgggaacagg ggcacctgga cggaggaacg cgggcgtctg     720
gaggggggac atcggctgag ctcagagcct ctcctccttc ctgtcccgag cttcccagca     780
actccgccac gttggagacc cggtacctgc gagagccagg gaactcgaga agtgcgtggc     840
cgaggctgct ttcctgaagg tgaatcattc tgtccaattg cctttcccca gagaacaaga     900
gggagggcgg gagagaggga acggaggatg tgtgagtgtg tgtgtgtgtg agagggagag     960
acgacgtgag gcgagaggaa aagtctttag tcggccttaa aagcaaacaa atcagagatg    1020
gaataagagc ggtaattgca gcaagaccgc cttgattctt gcagtccagg gagctgagcg    1080
caccgcgcgc atccggcgag gacaggaggc gaccgcgggc gctgccaagg gctgcgggac    1140
tttggctttt cctcagtaaa caaatctttt gattactttg acactgtgga ataaagaagc    1200
ggggagaagg atcaggctca ccttcacccg cttcaggggg attccagctt ggatgtcaga    1260
ttcctgaacc gtctttgcca tcggaggagg aaacacatcc acacacgcgc gcgcacactc    1320
```

gcacgctcag ggccacactc acacgccgcc ctccacgatc acacagcacc agacttcggt 1380
tccctatgcc cctggatggt gacggcggat tggcatcttg gaagcgatgc gagagcgata 1440
aggctggcgc cggcccgcaa aagctgcagg agcatcgcta ggtgttgccg ccaccgggaa 1500
gcggggctgc aggatgagta agagatactt acagaaagca acaaaaggaa aactgctaat 1560
aataatattt attgtaacct tgtgggggaa agttgtatcc agtgcaaacc atcataaagc 1620
tcaccatgtt aaaacgggaa cttgtgaggt ggtggcactc cacagatgct gtaataagaa 1680
caagatagaa gaacggtcac aaacagtcaa gtgctcctgc ttccctgggc aggtggcagg 1740
caccacgcga gctgctccat catgtgtgga tgcttcaata gtggaacaga aatggtggtg 1800
ccatatgcag ccatgtctag agggagaaga atgtaaagtt cttccggatc ggaaaggatg 1860
gagctgttcc tctgggaata aagtcaaaac aactagggta acccattaac ccaggagaaa 1920
tcaagtgatc ctcaaggctg atgacattga acatgcgcat agaaacttaa ctcaactcct 1980
gaggtgatct tgaagatttt tataccactt gaaagaggcg ctcaatagtc tatttccaag 2040
ggatttcatg gcctcttctt gaaatcaaga ctttttaaaa gtcagacatg aacttgcatg 2100
tcatgaagat ttcagcagat ttgaactgtg ttcaacttgt aaattgttaa aagaatttga 2160
agtcactgtc tgaggagctg gtgaagagtt gtttttttca gggtgatgtt agagacagtc 2220
acctttgag ttattggctc cagatgtgac tacttttctt gtttctgcaa gctgtatccc 2280
aagtgcactg tccttctgtc ctggatgtgt tcctgggtcc tatgttcatt tgctagtggg 2340
actacacatg gctttaatga catttccttt gagaactttt cctctggcat ggtgtagact 2400
gagacaattt tatttatatc ctaatcttgg agctcagaaa gcctacatgt tttaacatct 2460
taaagttgct tttgttaaag gaatggaaat atatatccat tggtaataat gttggcaagt 2520
aatagttatc tgaataaatc aatcatataa gaatgtatag acaagctgac atatttccct 2580
aaggctaaca acaccctgct gaagctcttt gtcaaatagg tagtagttag aactggattg 2640
ccattttcat tatataatac tttgtacctc tagagcactc tccctttctg ttttttttta 2700
agtgagcttt tctttaattt tttatgttta cttattccct tcacagaaat cagcagtgag 2760
cagtcaagtt aatgggtagc cttcagtttc aaaaaaattg acagggatgc atgtgagttt 2820
ctgatttctt agcttgaaca ttattcactt agatttcttc cagtattttt taaaaaactg 2880
tcctatctca ttttaaaaga ctttcttttg cttgatccca atgactgttt gaatgcttat 2940
atatttgttc aatctgttga tagaaaaaat tgttcatttt cctcagtctc aaatttataa 3000
atatttgctt acagttttcc tattcaaaca atttgttagg ccaatatttt gtgacatttt 3060
tgtagcgatt ttaacgttta tggttttggt tctacaggaa agtcataaat atttaaaggc 3120
cttaaacatg tatgtacttt ttttttctaa gttatagaat gtataatttt gtactacatt 3180
tattttgttt catttgtgat atgaagggag agaagaaaga aaagtgcata gccattctgt 3240
aacaatattg tgtaaaccta tagtttgaag gaatgcaagg agaaggattt ctgtgtttta 3300
ctcattttag gctgttcaga agatgcttca aaaattgtcc tgttagaatt tccatcatgg 3360

aaggtggtat ggaagaaggt atggaaatac tttgtattct aaaaactcac tgacgtggtc 3420
agttagacat acgttggttt ccaggataga ggcccatata tcctggggag ctttggtcta 3480
ttagtttgtg acaatattca aaggccaaaa cactactcag acactttcct gggaagagca 3540
actaaaaatg taaaattggt taaaaataaa atctgaaaag tatgtatctc acattgaact 3600
aaaatccact gtctcataag ttcatggaat gaaatggctt tctgcctcca ttttaatcat 3660
gcataaaatg aattagatgg ctttgagtgg attttcacaa tggctcaaga ctatatgaaa 3720
ttataaaaaa aaagttgccc tggggtttct gcatcaatta gaatatcatt aatttctttg 3780
taaccaagtg aaaaactata ctttttggaa attatgaatt tgtcctaggt ttgtttgaga 3840
tttgaaatta tacatcatgc ttctcatttt ttaaactatg ttctttaaat caacactgga 3900
aactctgtat tatatacaag tgtaatacat gcatataata gaaaaaaaac atggaatttc 3960
aaatatacta actagattat ccccagtaga ttaatgttgt gactattcag aaaaggtgaa 4020
taaaattggg atataaaatg gactctcttt cataaaaaaa aaaaaaaaa 4069

<210> 3
<211> 1077
<212> DNA
<213> sam3_cDNA

<400> 3

```
acttgtccag gtggagtgag tctgaggaca gcagatgaac agacagaaac tgaaagatcc      60
ccaaaaagga tgagtgagag ggtcgagcgg aactggagca cgggcggctg gctgctggca     120
ctgtgcctgg cctggctgtg gacccacctg accttggctg ccttgcagcc tcccactgcc     180
acagtgcttg tgcagcaggg cacctgcgag gtgattgcgg ctcaccgctg ctgcaaccgg     240
aaccgcatcg aggagcgctc ccagacggtg aaatgctcct gtttttctgg ccaggtggcc     300
ggcaccacgc gggcaaagcc ctcctgcgtg gacgcctcca tcgtcctgca gagatggtgg     360
tgtcagatgg agccctgcct gccgggggag gagtgtaagg tgctcccgga cctgtcggga     420
tggagctgca gcagtggaca caaagtcaaa accaccaagg tcacacgata gctcttgggg     480
gtcacggcct ggacaagaaa ggcttgactg agccgtgaac tgaagaatgg tatccagtca     540
tcagccagga aagatgggga ttcacttaca tgcctcatgt caaatgcagc atcagtcttt     600
ccggggcatt tcagttaagc tgctcagcag atatggatgg atctgcaatc acatacctaa     660
tgtggagctg ggcttttctg gagacacgaa ggtcaacaca caattcctgc ccttaaggaa     720
tgtccagttg aattggagag ttgatgacag acaatttaga taatttaggt taaagtactt     780
gataccagac tgcggcttct gggccacatg ctatggcatg atgggggttt gggaatgaga     840
ttcccacagt tcttcagata ccctgtggcc acagggcata gaaacaagag gtcacattca     900
gcacccacca cctccctctt tcgcatcagt tctgaatccc cagcaagctg ttaacatgtt     960
gcaggaaaac actctcccct tatgccagac cagcagtatc tcatttggat gggattggat    1020
tgacttgcgg aaggaaagta aaaataaagc caaataactt cccaaaaaaa aaaaaaa       1077
```

<210> 4
<211> 133
<212> PRT
<213> sam1_protein

<400> 4

Met Ala Met Val Ser Ala Met Ser Trp Val Leu Tyr Leu Trp Ile Ser
1               5                   10                  15

Ala Cys Ala Met Leu Leu Cys His Gly Ser Leu Gln His Thr Phe Gln
            20                  25                  30

Gln His His Leu His Arg Pro Glu Gly Gly Thr Cys Glu Val Ile Ala
        35                  40                  45

Ala His Arg Cys Cys Asn Lys Asn Arg Ile Glu Glu Arg Ser Gln Thr
    50                  55                  60

Val Lys Cys Ser Cys Leu Pro Gly Lys Val Ala Gly Thr Thr Arg Asn
65                  70                  75                  80

Arg Pro Ser Cys Val Asp Ala Ser Ile Val Ile Gly Lys Trp Trp Cys
                85                  90                  95

Glu Met Glu Pro Cys Leu Glu Gly Glu Glu Cys Lys Thr Leu Pro Asp
            100                 105                 110

Asn Ser Gly Trp Met Cys Ala Thr Gly Asn Lys Ile Lys Thr Thr Arg
        115                 120                 125

Ile His Pro Arg Thr
    130

<210> 5
<211> 131
<212> PRT
<213> sam2_protein

<400> 5

Met Ser Lys Arg Tyr Leu Gln Lys Ala Thr Lys Gly Lys Leu Leu Ile
1               5                   10                  15

Ile Ile Phe Ile Val Thr Leu Trp Gly Lys Val Val Ser Ser Ala Asn
            20                  25                  30

His His Lys Ala His His Val Lys Thr Gly Thr Cys Glu Val Val Ala
        35                  40                  45

Leu His Arg Cys Cys Asn Lys Asn Lys Ile Glu Glu Arg Ser Gln Thr
    50                  55                  60

Val Lys Cys Ser Cys Phe Pro Gly Gln Val Ala Gly Thr Thr Arg Ala
65                  70                  75                  80

Ala Pro Ser Cys Val Asp Ala Ser Ile Val Glu Gln Lys Trp Trp Cys
                85                  90                  95

His Met Gln Pro Cys Leu Glu Gly Glu Glu Cys Lys Val Leu Pro Asp
            100                 105                 110

Arg Lys Gly Trp Ser Cys Ser Ser Gly Asn Lys Val Lys Thr Thr Arg
        115                 120                 125

Val Thr His
    130

<210> 6
<211> 133
<212> PRT

<213> sam3_protein

<400> 6

```
Met Ser Glu Arg Val Glu Arg Asn Trp Ser Thr Gly Gly Trp Leu Leu
1               5                   10                  15

Ala Leu Cys Leu Ala Trp Leu Trp Thr His Leu Thr Leu Ala Ala Leu
            20                  25                  30

Gln Pro Pro Thr Ala Thr Val Leu Val Gln Gln Gly Thr Cys Glu Val
        35                  40                  45

Ile Ala Ala His Arg Cys Cys Asn Arg Asn Arg Ile Glu Glu Arg Ser
    50                  55                  60

Gln Thr Val Lys Cys Ser Cys Phe Ser Gly Gln Val Ala Gly Thr Thr
65                  70                  75                  80

Arg Ala Lys Pro Ser Cys Val Asp Ala Ser Ile Val Leu Gln Arg Trp
                85                  90                  95

Trp Cys Gln Met Glu Pro Cys Leu Pro Gly Glu Glu Cys Lys Val Leu
            100                 105                 110

Pro Asp Leu Ser Gly Trp Ser Cys Ser Ser Gly His Lys Val Lys Thr
        115                 120                 125

Thr Lys Val Thr Arg
    130
```

<210> 7
<211> 26
<212> DNA
<213> sam2_Forward

<400> 7
acgccgctga atgaaccgat taccgg 26

<210> 8
<211> 24
<212> DNA
<213> sam2_reverse

<400> 8
gagcgaacgc actgctttac acac 24

<210> 9
<211> 23
<212> DNA
<213> beta-actin_forward

<400> 9
gaggagcacc ccgtcctgct cac 23

<210> 10
<211> 23
<212> DNA
<213> beta-actin_reverse

<400> 10

gatggctgga acagggcctc tgg 23

<210> 11
<211> 20
<212> DNA
<213> Sam2cnu1_forward

<400> 11
tctactgagg agtggtgtga 20

<210> 12
<211> 20
<212> DNA
<213> Sam2cnu1_reverse

<400> 12
ggtcagtttc agagagctgg 20

<210> 13
<211> 12
<212> DNA
<213> left ZFN

<400> 13
tgctcctgct tc 12

<210> 14
<211> 12
<212> DNA
<213> right ZFN

<400> 14
caggtggcag ga 12

<210> 15
<211> 2248
<212> DNA
<213> sam4_cDNA

<400> 15

```
tgggctcggc tccgcactgc tagctgcgcg ccgccctgga cggggcgcac cgactgcgcg      60
cgcggctgcg ggcaaacatc gggagtcctg cctcagctgc cgcttctcca gcagcagctt     120
caggcttctc ccgcaggagc ttcgggcttc tcctggtaga gacgtgggaa cttttcttct     180
cctggcgagg ctgcagaggt gatgggccgc tcccggggct cccgcgggga ggcggcacgg     240
tgagcgtcct cgggctccgg tgcggcgatc agtacctagt tccggacgcg ccggtccgac     300
ttggatgccg gctctagtcg agtcttctga gctacgataa tttttggaa cggcagaaat      360
gattggttct agcaacagat gggaatttgg agtcactctg aaatatatcc tggaataagt     420
gtgtttgact agaaccacat cttatgaggt ccccaaggat gagagtctgt gctaagtcag     480
tgttgctgtc gcactggctc tttctagcct acgtgttaat ggtgtgctgt aagctgatgt     540
ccgcctcaag ccagcacctc cggggacatg caggtcacca ccaaatcaag caagggacct     600
gtgaggtggt cgccgtgcac aggtgctgca ataagaaccg catagaagag cggtcacaaa     660
cggtcaagtg ctcttgcttc ccgggacagg tggcgggcac aactcgggct caaccttctt     720
```

gtgttgaagc ttccattgtg attcagaaat ggtggtgtca catgaatccg tgtttggaag 780
gagaggattg taaagtgctg ccagattact caggttggtc ctgtagcagt ggcaataaag 840
tcaaaactac gaaggtaacg cggtagcgaa gagagaggtg tgcttcaatc ctggaggggc 900
agcaggaggc ggagctcttt tgcttggatt cccatcatgg cccctctgca gaaaattgtc 960
taggatttca gcaacttcat atttgtatat gtgagctgtg agaggtggca ttcacttaac 1020
tggcccagcc ctctctgctt cgtgatttta tttcattgaa ttataaccac aagccaccac 1080
ccatttgaca tcctctctgg attcccaagg agcatacctc caaaatccga gaagagcaaa 1140
tcagagtctt caaaatggat caccactaag ggcatgttca ttcttcactt tctttctgct 1200
tttacaaaag aacttggatg tatgttccaa agggtcctca ttctgttcct cttttgaact 1260
tttccttttg tccttgtatt aaagtggttt taaaggggtc taaaaagatt ttggcaaaac 1320
atatttgcag atgtagatta gctggtgaag aaaattactg ctagagatca actgattaac 1380
tggtaaagaa cgtttatttt ataacccttg aagaatagaa ggacatagtt ggattattgt 1440
gtgtgcattg tatttttact tctatttttt ttttgctttc cattttccag ttagcagaga 1500
taaaatgaga gcgttttaac ttcaatgtac cattttactg agtgctaagg aagcatatca 1560
attccaatat tttataacca aagctctatc agaacatatt tataaaactt gttggaattt 1620
ttacggcttt tgtgtagtca tgtaggtaaa tcatttaaaa tataaaacaa tctcaattta 1680
gatcaagggt tatttcttag atcaaattta tgccaattat atgaaaagat tttaactccg 1740
agacaggagt ctttcagtgc tgaattttta gactgtaaat gagttcttct taacttagct 1800
gtttccctac ttctgtgact tctgtgttag ccatcttatt tctttaaaat ctgagtcctg 1860
attggcttaa tgattttgca gcagacatgt ctccacatat tctcaaatgc tgtcatgcgg 1920
aaacgtatga aacagatgaa gaatgactga cccagatttt agatgtataa tgttgttaaa 1980
gtacatacta ctgtaaaaat atgggatgaa ttttatatat taagaaatgc caaaaacata 2040
gtttctgcac caagttaatt atccctgtcc tttcacattt ataggggaa aataaatact 2100
ttatagccta acagttattt gatgttattc ttgcagaggg aatggaaagg aatggaaaga 2160
tttgttggcg taatttttga atatttgtta tgatcatatg aataagtaaa aaaattcatc 2220
ctgctgatgg caaaaaaaaa aaaaaaaa 2248

<210> 16
<211> 2615
<212> DNA
<213> sam5_cDNA1

<400> 16.

gcggcggcgg cggaggatgg cgcgcgcggg gcccgcacgt ggaggccggc gcgggggcgc 60

gggcagggcc ggctgctgag acgcgctgct gcccccgcg cgggcgccgc ggcttcaatg 120

gcgccatcgc ccaggaccgg cagccggcaa gatgcgaccg ccctgcccag catgtcctca 180

actttctggg cgttcatgat cctggccagc ctgctcatcg cctactgcag tcagctggcc 240

gccggcacct gtgagattgt gaccttggac cgggacagca gccagcctcg gaggacgatc 300
gcccggcaga ccgcccgctg tgcgtgtaga aaggggcaga tcgccggcac cacgagagcc 360
cggcccgcct gtgtggacgc aagaatcatc aagaccaagc agtggtgtga catgcttccg 420
tgtctggagg gggaaggctg cgacttgtta atcaaccggt caggctggac gtgcacgcag 480
cccggcggga ggataaagac caccacggtc tcctgacaaa cacagcccct gaggggcccc 540
gggagtggcc ttggctccct ggagagccca cgtctcagcc acagttctcc actcgcctcg 600
gacttcaccc gttctctgcc gcccgcccac tccgtttccc tgtggtccgt gaaggacggc 660
ctcaggcctt ggcatcctga gcttcggtct gtccagccga cccgaggagg ccggactcag 720
acacataggc ggggggcggc acctggcatc agcaatacgc agtctgtggg agcccggccg 780
cgcccagccc ccgccgaccg tggcgttggc cctgctgtcc tcagaggagg aggaggagga 840
ggcagctccg gcagccacag aaggctgcag cccagcccgc ctgagacacg acgcctgccc 900
caggggactg tcaggcacag aagcggcctc ctcccgtgcc ccagactgtc cgaattgctt 960
ttattttctt atactttcag tatactccat agaccaaaga gcaaaatcta tctgaacctg 1020
gacgcaccct cactgtcagg gtccctgggg tcgcttgtgc gggcgggagg gcaatggtgg 1080
cagagacatg ctggtggccc cggcggagcg gagagggcgg ccgtggtgga ggcctccacc 1140
ccaggagcac cccgcgcacc ctcggaggac gggcttcggc tgcgcggagg ccgtggcaca 1200
cctgcgggag gcagcgacgg cccccacgca gacgccggga acgcaggccg ctttattcct 1260
ctgtacttag atcaacttga ccgtactaaa atccctttct gttttaacca gttaaacatg 1320
cctcttctac agctccattt ttgatagttg gataatccag tatctgccaa gagcatgttg 1380
ggtctcccgt gactgctgcc tcatcgatac cccatttagc tccagaaagc aaagaaaact 1440
cgagtaacac ttgtttgaaa gagatcatta aatgtatttt gcaaagccta aagttatata 1500
tttaacagtt tttatatgtt gtatatttgt agaaaatcct atttaacaat taacgtggca 1560
gtcccggccg tcctgagagt cgggccgagc cccgtgtgtt tctgaagact ctgggggtgg 1620
gacacggcgg ggaggtggtg ccccgcggac cccggggtgc caggcacgga aggcgggact 1680
ctgggagaag cgtgcggagg accgtggcgt cggcgtcccg gatgtgtcgg tcgtgcccgg 1740
ggaggccggg ttcccctcgc tgcgggccag gcttggctcc tgattccctc tctggtccct 1800
gtattggtca acacttgagc gtacaatatc ttgaacatgc ttcttccaat gggttttgtt 1860
tcccatttcc tgcccctttc gccactcacg gaccttgagg ccagttgacg gcccttctcc 1920
ccacgcctgt gtccccgcgt tctgagaagt cctctgtctt cgtgtcacta ggtccagaaa 1980
gtcgcgccgg gcagaggcgc aggcggggcc ggcagggccg aggaataagc gacaattctg 2040
gtttttctcc cctggccgtc gttcgccagc ctccttcatt ttcctgagtt cccgctgaag 2100
tatatactac ctatgagtcc aattaacatg agtattatgc tagttctatc ctactaaaaa 2160
aaacgtaaaa aaataactat atagaagctg ttccagcaac catagactga agatacgaaa 2220
gaaaatccat ttatttaaga cctgttccgg tatccatgag gacataattt acctttcagt 2280

caccacaaat ttataggcat ttgtatcctg gactaaaaga aggggctgag gttgggtttg 2340

tcatcacaga gggggtgggc ctggaaaggg tccttcccaa gctgccccgg ctccggcggc 2400

ccgggccggc agcctctgcc agccagcgtc ctcacggcct ccccctcgcc tgtttctttt 2460

gaaagcaagt gtagacacct tcgagggcag agatcgggag atttaagatg ttacagcata 2520

tttttttttc ttgttttaca gtattcaatt ttgtgttgat tcagctaaat tatgaaaaat 2580

aaagaaaaac tcctttgata aaaaaaaaaa aaaaa 2615

<210> 17
<211> 2652
<212> DNA
<213> sam5_cDNA2

<400> 17

```
aaagacacaa atcgcctccc ggagtggcgc ctccagtcgc ggcggagcgc ggcgttggcg      60
gcggatggag ggcgcgagcg ggcggccgcg gaggctgcac ccggcggggc gctgatgcgg     120
cgcctggacc ttcgctgcgc gacttcgggg gcgtcggccg agttgggact ccgcgatgca     180
gctcctgaag gcgctctggg cactggcagg ggccgcgctc tgctgcttcc tcgtcctagt     240
gatccacgcg cagttcctca aagaaggtca gctggccgcc ggcacctgtg agattgtgac     300
cttggaccgg gacagcagcc agcctcggag gacgatcgcc cggcagaccg cccgctgtgc     360
gtgtagaaag gggcagatcg ccggcaccac gagagcccgg cccgcctgtg tggacgcaag     420
aatcatcaag accaagcagt ggtgtgacat gcttccgtgt ctggaggggg aaggctgcga     480
cttgttaatc aaccggtcag gctggacgtg cacgcagccc ggcgggagga taaagaccac     540
cacggtctcc tgacaaacac agcccctgag gggccccggg agtggccttg gctccctgga     600
gagcccacgt ctcagccaca gttctccact cgcctcggac ttcacccgtt ctctgccgcc     660
cgcccactcc gtttccctgt ggtccgtgaa ggacggcctc aggccttggc atcctgagct     720
tcggtctgtc cagccgaccc gaggaggccg gactcagaca cataggcggg gggcggcacc     780
tggcatcagc aatacgcagt ctgtgggagc ccggccgcgc ccagcccccg ccgaccgtgg     840
cgttggccct gctgtcctca gaggaggagg aggaggaggc agctccggca gccacagaag     900
gctgcagccc agcccgcctg agacacgacg cctgccccag gggactgtca ggcacagaag     960
cggcctcctc ccgtgcccca gactgtccga attgctttta ttttcttata ctttcagtat    1020
actccataga ccaaagagca aaatctatct gaacctggac gcaccctcac tgtcagggtc    1080
cctggggtcg cttgtgcggg cgggagggca atggtggcag agacatgctg gtggccccgg    1140
cggagcggag agggcggccg tggtggaggc ctccacccca ggagcacccc gcgcaccctc    1200
ggaggacggg cttcggctgc gcggaggccg tggcacacct gcgggaggca gcgacggccc    1260
ccacgcagac gccgggaacg caggccgctt tattcctctg tacttagatc aacttgaccg    1320
tactaaaatc cctttctgtt ttaaccagtt aaacatgcct cttctacagc tccatttttg    1380
atagttggat aatccagtat ctgccaagag catgttgggt ctcccgtgac tgctgcctca    1440
tcgataccccc atttagctcc agaaagcaaa gaaaactcga gtaacacttg tttgaaagag    1500
```

atcattaaat gtattttgca aagcctaaag ttatatattt aacagttttt atatgttgta 1560
tatttgtaga aaatcctatt taacaattaa cgtggcagtc ccggccgtcc tgagagtcgg 1620
gccgagcccc gtgtgtttct gaagactctg ggggtgggac acggcgggga ggtggtgccc 1680
cgcggacccc ggggtgccag gcacggaagg cgggactctg ggagaagcgt gcggaggacc 1740
gtggcgtcgg cgtcccggat gtgtcggtcg tgcccgggga ggccgggttc ccctcgctgc 1800
gggccaggct tggctcctga ttccctctct ggtccctgta ttggtcaaca cttgagcgta 1860
caatatcttg aacatgcttc ttccaatggg ttttgtttcc catttcctgc ccctttcgcc 1920
actcacggac cttgaggcca gttgacggcc cttctcccca cgcctgtgtc cccgcgttct 1980
gagaagtcct ctgtcttcgt gtcactaggt ccagaaagtc gcgccgggca gaggcgcagg 2040
cggggccggc agggccgagg aataagcgac aattctggtt tttctcccct ggccgtcgtt 2100
cgccagcctc cttcattttc ctgagttccc gctgaagtat atactaccta tgagtccaat 2160
taacatgagt attatgctag ttctatccta ctaaaaaaaa cgtaaaaaaa taactatata 2220
gaagctgttc cagcaaccat agactgaaga tacgaaagaa aatccattta tttaagacct 2280
gttccggtat ccatgaggac ataatttacc tttcagtcac cacaaattta taggcatttg 2340
tatcctggac taaaagaagg ggctgaggtt gggtttgtca tcacagaggg ggtgggcctg 2400
gaaagggtcc ttcccaagct gccccggctc cggcggcccg ggccggcagc ctctgccagc 2460
cagcgtcctc acggcctccc cctcgcctgt ttcttttgaa agcaagtgta gacaccttcg 2520
agggcagaga tcgggagatt taagatgtta cagcatattt tttttcttg ttttacagta 2580
ttcaattttg tgttgattca gctaaattat gaaaaataaa gaaaaactcc tttgataaaa 2640
aaaaaaaaaa aa 2652

<210> 18
<211> 140
<212> PRT
<213> sam4_protein

<400> 18

Met Arg Ser Pro Arg Met Arg Val Cys Ala Lys Ser Val Leu Leu Ser
1 5 10 15

His Trp Leu Phe Leu Ala Tyr Val Leu Met Val Cys Cys Lys Leu Met
20 25 30

Ser Ala Ser Ser Gln His Leu Arg Gly His Ala Gly His His Gln Ile
35 40 45

Lys Gln Gly Thr Cys Glu Val Val Ala Val His Arg Cys Cys Asn Lys
50 55 60

Asn Arg Ile Glu Glu Arg Ser Gln Thr Val Lys Cys Ser Cys Phe Pro
65 70 75 80

Gly Gln Val Ala Gly Thr Thr Arg Ala Gln Pro Ser Cys Val Glu Ala
85 90 95

Ser Ile Val Ile Gln Lys Trp Trp Cys His Met Asn Pro Cys Leu Glu
100 105 110

Gly Glu Asp Cys Lys Val Leu Pro Asp Tyr Ser Gly Trp Ser Cys Ser
115 120 125

Ser Gly Asn Lys Val Lys Thr Thr Lys Val Thr Arg
130 135 140

<210> 19
<211> 132
<212> PRT
<213> sam5_protein1

<400> 19

Met Ala Pro Ser Pro Arg Thr Gly Ser Arg Gln Asp Ala Thr Ala Leu
1 5 10 15

Pro Ser Met Ser Ser Thr Phe Trp Ala Phe Met Ile Leu Ala Ser Leu
20 25 30

Leu Ile Ala Tyr Cys Ser Gln Leu Ala Ala Gly Thr Cys Glu Ile Val
35 40 45

Thr Leu Asp Arg Asp Ser Ser Gln Pro Arg Arg Thr Ile Ala Arg Gln
50 55 60

Thr Ala Arg Cys Ala Cys Arg Lys Gly Gln Ile Ala Gly Thr Thr Arg
65 70 75 80

Ala Arg Pro Ala Cys Val Asp Ala Arg Ile Ile Lys Thr Lys Gln Trp
85 90 95

Cys Asp Met Leu Pro Cys Leu Glu Gly Glu Gly Cys Asp Leu Leu Ile
100 105 110

Asn Arg Ser Gly Trp Thr Cys Thr Gln Pro Gly Gly Arg Ile Lys Thr
115 120 125

Thr Thr Val Ser
130

<210> 20
<211> 125

<212> PRT
<213> sam5_protein2

<400> 20

Met Gln Leu Leu Lys Ala Leu Trp Ala Leu Ala Gly Ala Ala Leu Cys
1 5 10 15

Cys Phe Leu Val Leu Val Ile His Ala Gln Phe Leu Lys Glu Gly Gln
20 25 30

Leu Ala Ala Gly Thr Cys Glu Ile Val Thr Leu Asp Arg Asp Ser Ser
35 40 45

Gln Pro Arg Arg Thr Ile Ala Arg Gln Thr Ala Arg Cys Ala Cys Arg
50 55 60

Lys Gly Gln Ile Ala Gly Thr Thr Arg Ala Arg Pro Ala Cys Val Asp
65 70 75 80

Ala Arg Ile Ile Lys Thr Lys Gln Trp Cys Asp Met Leu Pro Cys Leu
85 90 95

Glu Gly Glu Gly Cys Asp Leu Leu Ile Asn Arg Ser Gly Trp Thr Cys
100 105 110

Thr Gln Pro Gly Gly Arg Ile Lys Thr Thr Thr Val Ser
115 120 125

<210> 21
<211> 436
<212> DNA
<213> sam1acDNA_Z

<400> 21

cagccggtgc tcctgccatg tcctggctcc tgtgtctgtg gataagtgtt agctgcttac 60
tgctctgtca cgccacttta tacgaaacca tccagcagca tcatgtgacc cgtccgggcc 120
gaaacgccat ccagatactg gaaggaggaa catgtgaggt gatcgcagca caccgatgtt 180
gcaacaagaa ccgtattgag gaacgttccc agacggtcaa gtgttcatgt ttaccaggaa 240
aagtggctgg cacgacacga aacaaaccct cctgtgtaga cgcctccatt gtgattggga 300
agtggtggtg tgagatggag ccatgtctgg agggagaaga gtgcaagacg ctaccagaca 360
actccggctg gatgtgttcc tctgggaata agatcaagac cacgaggatt catccccgga 420
cctaacaaag agctcc 436

<210> 22
<211> 883
<212> DNA
<213> sam1bcDNA_Z

<400> 22

gggcgattcg atatcgcggc cgcgatccga cgaaagtgag aagacagaag aactgagagt 60
ttctacaaga cagcaatacc tggacgaaag gatatccgga cacctcacaa tgaatggatt 120
gaggacaaag tgttgagttt tacatacagc tgtccctgga cgatacagag gagtgttggg 180
atgtcgtggc tgctgtgggt ttgggtcagt ctgacagtcc tgctgatgtg tgagggcact 240
ttctatgaga ccatccagca gcacctggca ccgcctggaa ccaacataca gatcctcgaa 300
ggagggacat gcgaggtgat cgcggcgcac aggtgctgta acaaaaacaa gattgaagag 360
agatctcaga cagtgaagtg ctcctgttta cctgggaaag tggcaggaac caccaggaac 420
aaaccctcct gtgttgacgc ctccattgtg attgggaagt ggtggtgtga gatggagccg 480
tgtctggagg gagaagagtg taagactcta ccagacaact ccggctggat gtgctactct 540
ggaaacaaga tcaaaaccac caggaatacc cacccataca catccttgta gcagaggtag 600
aatcactgca aagctcaaaa tggaggaact ttgaatgaag ggaaacatcc aagaggacaa 660
gctgaaataa gttttcccaa ctctgaccca acaaccatgt gtctaaagac ttgaaatgga 720
atcctcggct cataaatcca tctgtctctg agtaacacgg acaacagact ctttaagaaa 780
gtcgttgtgg gcgtgaacac tgctggctct tcttccctct gtttttgttt gacagctgtc 840
aatattgttt cctctcagtg gataattaaa ctcgtatccc ttt 883

<210> 23
<211> 505
<212> DNA
<213> sam2cDNA_Z

<400> 23

acgccgctga atgaaccgat taccggatcg atgaaccccc gagtcccgct ggactgatcg 60
ggctgatcgg atggattgat caggagttaa tcagagctgc aggatgagtg tccgagatct 120
tcttctgctt ctctctcaga ctcttcttct gcttctgctt ctcctgagat cttcagctca 180
ctccacagag cttcacattg ttgaggtgcg gacgggcacc tgtgaggtcg tgtctttaca 240
tcgctgctgc aacaagaaca agatcgagga gcgttcgcag accgtcaagt gctcctgctt 300
cccgggacag gtggcaggaa caaccagagc cgccccgtcc tgcgtcgacg ctgctatcgt 360
ccagcagaag tggtggtgtc agatgcagcc gtgtctggat ggtgaagagt gtaaagttct 420
gccggatctg aaaggatgga gctgcgccac cggaaacaag atcaagacca ccaagatctc 480
tccagaccga gatcagctgt ggtga 505

<210> 24
<211> 1580
<212> DNA
<213> sam3acDNA_Z

<400> 24

```
cgcttcaagt gctgaacact tgaggggcga gtcgagctcc tcaacgccgc tctcagtctc      60
cacgcagcct gaacacaaga ctacagcagc caaaaacacc actggtccat catggataag     120
gggatattgc cgatttgaaa ccttcgttat aatacccgca attaagggtc tcgtctttac     180
gtgcttattc agatccgttc cggcgcgcaa agttcagacg catctgaagg aactttggag     240
agctggagtt cagaggactc aatcgagact tttctgaact gaagcaggaa tcgctttaat     300
accatttgca ttctcgtttg tttgagtctg taggtgatgc ttttcctggg atcgtgtttg     360
cgcgaaggga aattatcagg ctttgccact gccggggttt tacttctgac aatgaagagc     420
aaatgagagc agtattactt cacctttatt gacagctcaa cgacaggatg cgggagaggg     480
agctgcagcg tgtgggcagg agattgctgc tgttgggcat ctgtctgctg tctctctggg     540
gtcagttcag cctggcctcg acacatcgca gccatattgt gcacgtcaaa gcgggaacct     600
gtgaggtcat tgctgcccac cgctgctgta acaagaacaa aatcgaggag cgctcccaaa     660
ctgtcaagtg ctcctgcttc cctgggcaag tggctggaac aacaagagct gctccatcct     720
gtgtggacgc atctatagta gcgcagaagt ggtggtgtca gatgcagccg tgtatggacg     780
gtgaggagtg taaggtgttg ccggatctaa aaggctggag ctgcagcaca ggcaacaaag     840
tcaagacgac caaggtcaca cgatagtctg ggaccaacat acatttggga ctagcagcat     900
tttttttctc tctccccatt ttttcgatca aagcaatgga aatgaagata catagacttc     960
aaactacttc acaactatcg ggatcaactt gttggacctc aactgaatac taatgtcact    1020
tggaatagac catttaaaca catggagtgt tttctctctc ttccccttgt cctcttctac    1080
agtattttca ctctgccaga ctcctgtctt ttgttttata aaagtgtcgg acaagatcat    1140
acgccatgta aagtaaatat tacatttaaa aaaaatcagt tcagagacaa ttctgccacc    1200

atggattata acggatttcg tttttcaagc gaaagtggac tcgagaggat cacttattac    1260
ttaaatcata tgttccagag gcactaatat tcattccact atccttcagt gccaatattt    1320
tctttcttct actatgagca gaatttttga tcagtgtgag gtgtatgttt ttttggctat    1380
ggttttgaaa cgggacttgt gcaaaaacaa atcgcaagtg ttatgataag aattacaggt    1440
attaaacctg ataaaatgtg tacgggagct gtaaaaaaga tggcaaaaaa gatttattgt    1500
ttgtttttaa aagccttggt ggccaatatt gagtcaaagt gtcagtatta tctcaaataa    1560
aactctatct ctatggaaaa                                                1580
```

<210> 25
<211> 1728
<212> DNA
<213> sam3bcDNA_Z

<400> 25

```
caggtttaga cgagtcttaa ggctactcag gtggccctgt gcatttagat ggcagaatta      60
atgcaagtca ggtgaacctg taaaatattc agaaacaacg tgagctcaga aagagaagaa     120
gctgcaggac agctgtcaca aacgccgctg agtggcgctg attcaccgct cattgcgttt     180
cttcagaaca tccctggaga gcacagggat cgagtcagcg cagcgcagct ccagaccttt     240
gccttcagca ccggcagatc ctgcacagag cgcgaacgcg tctccagctc ggacaaacac     300
taggaaaaca agcccaaggg gatactacgc gagatgaggg ctccgttaca aacatctgtg     360
tgatttttaa accaggagcg aaatcgttga tgtgaagttt cacgcagctc tccgttgagt     420
tgagatgaag atctgagccg ctgtgagaga aggaactttg cgtcttcgcg cacccggacg     480
ccctgcgggg acatcgggcg cttcagtgat aagaggactg aacttctttt ctgtatttgg     540
ggctttctag cttactctga cagagtagac acagaaggga tcctttggtg cggattgttg     600
aagcatctcc agttcgtgga tggaataaat cataatggaa gggcaatgag acctctttct     660
gctgctgggc aataatccgg actaccgcgc caacaggatg caggagcggg cgctgcagag     720
ggcgtttgcg tggatcgtac tggtattttt ggcccttgtg ttgttttggg gtcatttgtc     780
tgaagcgtct tctcaccgca gtcacatcgt gcacgtgaaa gctggcacat gtgaggtgat     840
tgcagcccat cgctgctgta acagaaataa gattgaggag cgatcgcaga cggtcaagtg     900
ttcctgcttc cccggacaag tggctggaac gacgagagct gcaccctcct gtgtggacgc     960
atctatagta gctcagaagt ggtggtgtca aatgcagcca tgtgtggatg gtgaggagtg    1020
taaagtcctg cccgacctca caggctggag ctgcagcacg ggcaacaagg tgaagacaac    1080
caaggtgact cgatagttcc aagcaacaat gtcctgggac aaaagagctg aaaatggaga    1140
ctaactagag gcagcattga gcttcagcta acaggattta tcagtattag tcatatgaag    1200
attatctgca atcaggaaca gactgatctc ctccataaac attaggacca tcctagagtt    1260
aatgtgccct tctcaatgga ccccctggcc ttccggacgc tcgatctttc gaatgtggat    1320
caatagacgc tggaatgtgt tggctagggg gtcttgatgt tcatatgcag tttatttgtt    1380
ttttacagaa ggtttggatc acctacaaca gtgcagccca gtggagttta agctcttcag    1440
taacatgctc tccgtcattg tatttcagtt ctccccacaa ttccatgatt atccctgctc    1500
tacctgacca taaggtccaa ttattccttg tggaatcagc atttaaacgt ctggacctct    1560
aatggactca tgtgtatagt ctttatttct aaatgaaaag cttttcagcc ccacagtctg    1620
aaacaacaga ttagatgtac aagtatattc tagataatgt aaaagtgtaa aagatcaaag    1680
tgttattctc aaaaacaagc gaaataaagt ttaacggtta tcactttt                 1728
```

<210> 26
<211> 863
<212> DNA
<213> sam4cDNA_Z

<400> 26

aaaagaaagt aagctcgttt aaaacaaaca tctgtcttca ggactcctgc tacatcccga 60
agcgaaggag aatgctttcg ctttcataaa cggaggatct gagcggtgtt tgaggacaca 120
ggaggagaga catgtccagc tcacagagaa gagactgatc agacaacaca gaaagatgca 180
gctgtggggt cgatctctgt ggccgctgcg gattgtgctc ttgttcacct tggtaatgtg 240
ttcgtgtggg ttggtttctg cgggcacgcg gagtctgcgg ggacacacag cttcatacca 300
ggtaaagcag ggtacctgtg aggtggttgc catccaccgc tgctgcaata agaacaagat 360
tgaggagcgt tcacagaccg tcaagtgctc atgtttccct ggtcaggtgg ccggtaccac 420
acgtgcacag ccatcttgtg tggaggcttc catcgtgcta cagaagtggt ggtgccaaat 480
gcacccatgt ttggatggcg aggagtgcaa agctcttcca gatctgactg gctggtcctg 540
cagcaccggt aacaaagtca aaaccactaa ggtcacacga tagcaacaac atggcagttc 600
taccatgagg cccaagactc cttaacgacc cactggaaaa gaaaactttc ttgtttaaga 660
cactgacgtt gctggcgttt ttccataggc tccgcccccc tgacgagcat cacaaaaatc 720
gacgctcaag tcagaggtgg cgaaacccga caggactata aagataccag gcgtttcccc 780
ctggaagctc cctcgtgcgc tctcctgttc cgaccctgcc gcttaccgga tacctgtccg 840
cctttctccc ttcgggaagc gtg 863

<210> 27
<211> 1673
<212> DNA
<213> sam5acDNA_Z

<400> 27

acagacggag gcggagatac agacacggcg tttctccaag aacacacgcg tctcgtttgg 60
gaccttttac acgaccgttc gaaataatgc gggaattacg gctatttcga agctgacttt 120
atctgaatgt aggctttaca gcaagcgctg tcgacaggct actgtacaga aaacagttca 180
cagatcgcgc agttctccca gtgcgtaaaa cagacggaca ggatggtcgc gcatcacttc 240
acttagatcc acgcaacagc tacagacaaa ctggaaattt cagctccttt ttttctccga 300
tggaagctct aaaatataaa atatgttatt ttcctaaagt ttctattttt tgaaagaaaa 360
cggattgctt tctttctgaa tcttcaaacg aaaacgtagc agaggtgttg gttgttgtat 420

ggcattagaa tggagatgtg gcgtggatgc tgaaggcagt caggatgctg atgctgagag 480
ttgcgtgggc tctagcggga gcggcggtct gctgttttct catcgttctc atccactctc 540
gcttcctaag agacggtcaa cttgcagcag gcacctgtga gattgtcact ttagacaaag 600
acagcagtca gccacgcagg acgattgcca gacagacagc tcgctgtgca tgtaagaaag 660
gacagattgc tgggacaacg aatgccaggc cggcctgtgt ggacgctcgt atcgtgaaga 720
ccaagcagtg gtgtgacatg gtgccctgtc tagaggatga agagtgtgac ttgttagtaa 780
ataaatctgg ctggacttgt acacagccaa gtggcagagt gaaaactaca acggtgtcct 840
aacagtggga ggggttcctg gagcgctacc agacaaggcc tcttgcagat ttgcccacct 900
gctgtagata cttgctatgt ctcatcggct tgacacctgc ctctgcctac cagtctgcgt 960
ctcagctttg taaatgggca gtaccccctt gtgccacgga gagcatacta gatgttcaca 1020
aagcgcctca tggacagcag cagccttgag gaatttatga gcgactctga ggcacctacc 1080
ctgtttgaga ctgaaccaaa cataggcttt tttgggactc caccagcggt tctgacctag 1140
actgtgaacc aagccgcttt ttgaactgct ttctgctgaa gtacattttt gccactcatt 1200
tttacagacc aaagaggaaa aaataatgat ataatacaga aatttaaaaa aatccattga 1260
ctatgttagc acggactgag agacagactt gaaacgatta agacggtgaa aaaaatatat 1320
atatacctaa atgtagagag gtagatatag atctatgaag actttgctat acagcggcgg 1380
tctacaagca aaaatgctga catggaacac tgaaacgact gggttgacca ttcccattta 1440
tcacaatctg atgagaacct ttaatatttt acagatttcc ttgaaaggaa ctacccactg 1500
catttcaagg acagctagga tatagatact gtacacaatc acatcaacaa atttgtttag 1560
cattttttcag tagcaacatt ttggacagtt tgcattcagc atggaaggtt aaatggaacc 1620
gtaccccatt gtttatcgtc agaagatatt aaacaagaac atgaaaactg tca 1673

<210> 28
<211> 408
<212> DNA
<213> sam5bcDNA_Z

<400> 28

acgcgtcaga tcctcggaaa gatgcagcat ctccgtgtgg cgtgggcgct cgcgggagcg 60
gcggtgggct tttttctgct cttcttgatc caagcgcatt tcttcggcga aggtcagctg 120
gcggcaggta catgtgaaat cgtgactctg gaccgggaca gcagccagcc aagaaggacc 180
atcgcccggc agacggccag gtgcgcctgc aggaagggcc agattgcggg cactaccaga 240
gccagtccag catgtgtgga tgcgcaaatt gtcaggacca gacagtggtg tgagatgatg 300
ccttgtttgg atgacgaagg ctgtgaccta ttggtaaaca gaacaggctg gacgtgtgca 360
cagccaggag gccgagtgaa aacaaccacg gtatcctgac gccagtgg 408

## Description

## TECHNICAL FIELD

The present disclosure relates to a novel samdori2 (Sam2) gene, a composition including the gene for diagnosing, preventing or treating a mental disease, and a method for screening an agent for diagnosing, preventing or treating a mental disease using the gene.

## BACKGROUND ART

It has been known that one out of four persons in world population has a mental disease or a neurological disease in one's life. Also, it has been reported that incidence of a mental disease in KOREA is 31.4% (per a lifetime) and 19.3% (per a year). It has been also reported that 3 mental diseases occur in all age groups and 6 mental diseases occur in the age of between 15 to 44 (WHO, 2001). Depression is a fourth main cause of the whole diseases and has been assumed to become a second cause following ischemic cardiac diseases in 2020.

The technique to treat mental diseases has been greatly improved for decades, but peoples do not escape from suffer of mental diseases. As a representative example, for schizophrenia and depression, which are major mental diseases, at least one third of the patients consistently have symptoms which are not alleviated even by the current therapy. For remaining cases in which symptoms are alleviated, a considerable ratio of patients has decreased quality of life due to residual symptoms and functional disorders, which becomes a social and economical burden factor. In particular, for schizophrenia, the medicinal therapy, which is a current first-line therapy, is effective in positive symptoms, but less effective in negative symptoms or improvement in cognitive function. Therefore, development of a therapeutic agent having improved efficacy is urgently required.

Depression, i.e., depressive disorder, refers to a disease which shows decline in enthusiasm and depressed feeling as main symptoms and causes various cognition, psychological and physical symptoms, thereby causing decline in abilities to function in daily life. Incidence of depressive disorder per life time is 15%, and particularly about 25% in women, and depressive disorder is a serious disease causing changes in emotion, thinking, physical status, and behaviors. Major depression is not caused by one reason. Rather, emotional disorder occurs by interaction of various types of genes, epigenetic influence, embryological causes, and environmental influence which complexly damages emotion.

Further, anxiety refers to a widely very unpleasant and vaguely uneasy feeling and involves physical symptoms such as heart palpitations and sweat and behavior symptoms such as hypersensitiveness and hovering. The number of patients receiving specialized psychotherapy in hospitals due to depression or anxiety has been increased every year. Moreover, sales of antianxiety drugs are increased by about 200% in 2002. Therefore, it is no exaggeration to say that depression or anxiety takes most parts of mental diseases of contemporary peoples, and many people are suffered from depression and anxiety (Robert. F.Scmidt. Human physiology, p366; Min Seonggil. Latest Psychiatry, pp238-240; S. V., Pharmacol. Ther., 88, pp 213-227, 2000). When being anxious, the entire brain is in an arousal state, so that disorders occur in behavior of peripheral, an autonomic nervous system, sensation, perception, etc. Examples of organs associated with this function include brains such as a cerebrum limbic system (in particular, hippocampus, and cingulate gyrus), cerebral cortex (in particular, frontal lobe and temporal lobe), hypothalamus, ascending reticular system, and pituitary gland and peripheral organs such as thyroid and adrenal cortex. According to the recent brain imaging study, it has been known that anxiety is associated with disorders in the right hemisphere, frontal lobe, temporal lobe, and occipital lobe (Robert. F. Scmidt. Human physiology, p366; Min Seonggil. Latest Psychiatry, pp238-240).

After it has been demonstrated that iproniazid, which has been developed as an antituberculous agent, has the antidepressive effect as a monoamine oxidase inhibitor, development of recent antidepressants is invigorated. Chlorpromazine, which is an antipsychotic drug, has been developed as an antihistamine agent in early 1950s, and demonstrated to have an effect in treatment of schizophrenia. Similarly, imipramine has been synthesized as an antihistamine agent by Geigy Drug Co. (Swiss) in 1955. Therefore, the development has been invigorated. Through these findings, tricyclic antidepressants (TCAs) have been developed, and many additional TCAs such as nortriptyline, doxepin, and clomipramine, as well as amitriptyline and desipramine which have modified tricyclic structures are used. Then, second and third generations of antidepressants have been developed. In early 1980s, tetracyclic compounds which have similar structures and efficacy and are referred to as heterocyclics such as maprotiline and amoxapine have been commercialized. In 1980s, prozac, which is a selective serotonin reuptake inhibitor (SSRI), has been developed and used as a good therapeutic agent. However, it has been reported that prozac has side effects as follows: thirst, constipation, dysuria, visual impairment, and impotence which are anticholinergic actions, are shown; blood pressure, pulse, and cardiac conduction are significantly affected due to the action on the cardiovascular system; and orthostatic hypotension occurs due to block of the 1-adrenergic receptor. Also, it has been reported that the sedation effect is exhibited due to the antihistamine effect. Examples of recently used antidepressants include selective serotonin reuptake inhibitors, serotonin norepinephrine reuptake inhibitors, selective norepinephrine reuptake inhibitors, dopamine and norepinephrine reuptake inhibitors (DNRIs), norepinephrine and serotonin receptor antagonists (NaSSAs), serotonin receptor antagonism and serotonin reuptake inhibitors (SARIs), selective serotonin reuptake enhancers (SSREs), etc.

Since the antidepressants recently used in the treatment show low remission rate, sufficient therapeutic effects may not be obtained through recent drugs, so that it has been estimated that further antidepressant market would shrink. To overcome this, development of new concept of a therapeutic agent which overcomes current low remission rate is required. Antidepressants recently used in the treatment have representative side effects such as sexual dysfunction and weight gain, so that it has been expected that development of a new antidepressant which overcomes such side effects opens a new market. One of other key problems of antidepressants recently used in the treatment is that there is lag-time of several weeks or several months until the drugs show efficacy after administration.

Emotional responses such as fear and anxiety serve an important role in decision making and survival. The habenular nucleus is an area of brain which is located at the top of forebrain and maintains high homology in evolution. It has been known that this brain area controls behavior responses to stress, anxiety, and fear, and it has been particularly known that, in the human, functional disorders in the habenular nucleus is associated with depression, post-traumatic stress disorders, and schizophrenia (Hikosaka, Nat. Rev. Neurosci, 11, p 503-513, 2010; Amo, R. et al, J. Neurosci, 30, 1566-1574, 2010). Also, it has been found that, through the experiment of removing a habenular nucleus tissue in a zebrafish and mouse, the habenular nucleus controls fear and anxiety responses. However, understand and study about a molecular mechanism of the habenular nucleus in regulation of emotion is inadequate.

Therefore, the present inventors recognize the problems of typical technology in the prevention and treatment of mental diseases such as anxiety and depression, and have tried to develop a new neuromodulator. Consequently, a novel chemokine-like samdori2 (Sam2) protein expressed in a habenular nucleus is identified. Borg et al. (Hum. Genet. 2005; 118: 267-275) discloses that potential genes responsible for intellectual deficiency disrupted as a result of patient's chromosomal rearrangement map at12q14.1 (TAFA2),12q23.1 (METAP2), and 11p14.1(BDNF). To investigate the function of the Sam2, a Sam2-specific knockout zebrafish, which is prepared by using zinc finger nuclease (ZFN) gene scissor technology, is used for novel tank, scototaxis, and social cohesion tests. Therefore, it has been found that a considerably higher anxiety behavior than the control is shown; inhibitory neuron expression in neurons overexpressing Sam2 gene is decreased; and a patient due to Sam2 gene loss exhibits autism and a hyperanxiety symptom. Consequently, it has been found that the samdori2 can be used to develop a pharmaceutical composition for preventing and treating a mental disease, in particular, depression and autism, and therefore the present invention has been completed.

## DISCLOSURE OF THE INVENTION

### TECHNICAL PROBLEM

One object of the present disclosure is to provide a novel samdori2 gene, a composition including the gene for diagnosing, preventing or treating a mental disease, and a method of for screening an agent for diagnosing, preventing, or treating a mental disease using the gene.

### TECHNICAL SOLUTION

The invention is as set out in the claims. In order to achieve the objects, the present disclosure provides a samdori2 (Sam2) gene having any one of base sequences of SEQ ID NOS: 1-3 and 15-17.

The present disclosure also provides an expression vector including the samdori2 gene having any one of base sequences of SEQ ID NOS: 1-3 and 15-17.

Further, the present disclosure provides a transformant obtained by transforming a host cell with the expression vector including the samdori2 gene.

The present disclosure also provides a method for preparing a recombinant transformant expressing samdori2, the method including:

1) preparing an expression vector including the samdori2 gene; and
2) transforming a host cell with the expression vector.

Further, the present disclosure provides samdori2 protein encoded by the samdori2 gene.

The present disclosure also provides samdori2 protein having any one of amino acid sequences of SEQ ID NOS: 4-6 and 18-20.

Further, the present invention provides a pharmaceutical composition for preventing and treating a mental disease containing the expression vector including the samdori2 gene or samdori2 protein.

The present disclosure also provides a health food for preventing and alleviating a mental disease containing the expression vector including the samdori2 gene or samdori2 protein.

Further, the present disclosure provides a kit for diagnosing a mental disease including an antisense nucleotide for the samdori2 gene, a primer set or probe, or an aptamer or antibody for the samdori2 protein encoded by the samdori2 gene.

The present disclosure also provides a method for measuring an expression level of the samdori2 gene for monitoring a mental disease or providing information for diagnosis, the method including:

1) preparing cells isolated from a subject-derived habenular nucleus;
2) measuring an expression level of the samdori2 gene having any one of base sequences of SEQ ID NOS: 1-3 and 15-17 in cells of step 1) according to the present disclosure and
3) determining that a mental disease occurs or a risk is increased, when the samdori2 gene of step 2) according to the present invention is deleted or decreased compared to the normal control.

Further, the present disclosure provides a method for screening an agent for preventing or treating a mental disease, the method including:

1) treating cells expressing the samdori2 gene having any one of base sequences of SEQ ID NOS: 1-3 and 15-17, or the samdori2 protein having a base sequences of SEQ ID NOS: 1-3 and 15-17 with a sample;
2) measuring activity or expression of the samdori2 gene or samdori2 protein in step 1) according to the present invention; and
3) screening a subject sample which increases activity or expression of the samdori2 gene or samdori 2 protein in step 2) according to the present invention.

The present disclosure also provides a model animal for a mental disease in which the samdori2 gene having any one of base sequences of SEQ ID NOS: 1-3 and 15-17 is knocked out in a habenular nucleus of an animal other than a human.

Further, the present disclosure provides a method for preparing a model animal for a mental disease, the method including:

1) microinjecting a vector expressing a gene scissor specific for the samdori2 gene having any one of base sequences of SEQ ID NOS: 1-3 and 15-17 into a fertilized egg of an animal other than a human;
2) raising the animal in step 1) according to the present invention until the animal becomes an adult, and then breeding the animal with a wild type animal; and
3) screening an animal in which the samdori2 gene is knocked out among the bred animals in step 2) according to the present disclosure.

## ADVANTAGEOUS EFFECTS disclosure

The present invention relates to a novel chemokine-like samdori2 (Sam2) gene and use thereof. By constructing a Sam2-specific knockout zebrafish (wherein, the Sam 2 is expressed in a habenular nucleus) and finding that, through novel tank, scototaxis, and social cohesion tests, the zebrafish shows significantly higher fear and anxiety behaviors than the control; inhibitory neuron expression in neurons overexpressing Sam2 gene is decreased; and a patient due to sam2 gene loss shows autism and the hyperanxiety symptom, the Samdori2 can be usefully applied to development of a pharmaceutical composition for preventing and treating a mental disease associated with a habenular nucleus, in particular autism and depression.

## BRIEF DESCRIPTION OF THE DRAWINGS

FIGS. 1 and 2 are images showing a SAM gene amino acid sequence which is conserved in vertebrates.
FIG. 3 is an image showing a specific expression pattern of SAM gene family in a central nervous system of a zebrafish.
FIG. 3 a-h': identification of a samdori gene family expression pattern in a brain of a zebrafish after 3 days of fertilization.
FIG. 3 i-j': identification of SAM3a and SAM3b gene expression in a spinal cord of a zebrafish after 24 hours of fertilization.
FIG. 4 is an image showing an expression pattern of Sam2 gene-expressing cells using a marker.
FIG. 5 is an image showing SAM gene family expression pattern in mind bomb (mib).
FIGS. 6a and b are images showing that the Sam2 gene, among SAM genes, is consistently expressed from early development to the adult phase.

FIGS. 6c, d, e, and f are images obtained by observing development of efferent axons in a Et(-1otpa:mmGFP)hd1 transformed Sam2 knockout zebrafish.

FIG. 7 is an illustration showing Sam2 gene knockout fish preparation using ZFN, i.e. gene scissor technique.

FIG. 7a is an illustration showing gain of mutation in two alleles in which bases of 5 Sam2cnu1 and 17 Sam2cun2 are lost.

FIGS. 7b-b' is an illustration showing genotype separation of two alleles by using mutation-specific restriction enzyme (Sam2cnu1) and PCR (Sam2cnu1).

FIGS. 7c-c' is an image showing normal developed embryos of the control and Sam2 mutant after 3 days of fertilization.

FIG. 8 is an image showing an influence of Sam2 gene loss on a particular neuron development in a Sam2 gene knockout zebrafish.

FIGS. 8 a-a' and b-b' are images showing comparison of dopamine neuron marker expression patterns of the control and Sam2 gene knockout zebrafish.

FIGS. 8 c-c', f-f', and l-l' are images showing comparison of serotonin neuron marker expression patterns of the control and Sam2 gene knockout zebrafish.

FIGS. 8 d-d' is an image showing comparison of oxytocin neuron marker expression patterns of the control and Sam2 gene knockout zebrafish.

FIGS. 8 e-e' is an image showing comparison of interpenduncular nucleus expression patterns of the control and Sam2 gene knockout zebrafish.

FIGS. 8 g-g', h-h', i-i', and j-j' are images showing comparison of various neuron marker expression patterns of the control and Sam2 gene knockout zebrafish.

FIGS. 8 m-n' is an image obtained by observing nervous circuits of the control and Sam2 gene knockout zebrafish by using a transformant (Tg[hcrt:EGFP]) in which LC branch axons are connected in a hypothalamus.

FIG. 9 is an image showing a novel tank test result by using a Sam2 gene knockout zebrafish and control.

FIG. 9a is an image showing a moving distance of a single individual of the Sam2 gene knockout zebrafish and control.

FIG. 9b is an image showing an average speed of the Sam2 gene knockout zebrafish and control.

FIG. 9c is an image showing abnormal behaviors of the Sam2 gene knockout zebrafish and control.

FIG. 9d is an image showing freezing bouts of the Sam2 gene knockout zebrafish and control.

FIG. 10 is an image showing increase in anxiety by using a Sam2 gene knockout zebrafish through an open tank and scototaxis tests.

FIG. 10a is an image showing analysis and comparison after placing the control and Sam2 gene knockout zebrafish in an open tank for 30 minutes to measure thigmotaxis, and then measuring time for staying at the corner and center at one minute interval.

FIGS. 10b and c show the scototaxis test using the sam2 gene knockout zebrafish, and time for staying at white parts by tracing movement of the control and Sam2 gene knockout zebrafish is analyzed and compared.

FIG. 11 is an image obtained by measuring fear and anxiety through observation of a behavior index of a Sam2 gene knockout zebrafish.

FIG. 12 is an image obtained by measuring anxiety responses in group behaviors through observation of social cohesion of a Sam2 gene knockout zebrafish.

FIG. 13 is an image showing the anxiety response of a Sam2 gene knockout zebrafish by using a molecular marker.

FIG. 14 is an image showing an influence of a human Sam2 gene on excitatory and inhibitory synapses through culture of rat hippocampal neurons.

FIG. 15 shows an image investigating excitatory neuron molecular marker expression through Sam2 gene overexpression.

FIG. 16 is an image investigating autism and a hyperanxiety symptom due to Sam2 gene loss caused by microdeletion in a human.

## MODE FOR CARRYING OUT THE DISCLOSURE

Hereinafter, the present disclosure will be described in more detail.

The present disclosure provides a samdori2 (Sam2) gene having any one of base sequences of SEQ ID NOS: 1-3 and 15-17.

In a particular variant of the present disclosure, to investigate the samdori2 gene expression in a habenular nucleus, the present inventors perform reverse transcription polymerase chain reaction (RT-PCR) for isolating the samdori2 gene from wild type, *mind bomb* mutant, and Et(-1otpa::mmGFP)hd1 transformed zebrafishes. To investigate evolutional relationship between the gene isolated from the zebrafishes and a genome of a human, similarly on chromosomes is compared by using Synteny database (http://teleost.cs.uoregon.edu/acos/synteny_db/), NCBI (http://www.ncbi.nlm.nih.gov/). Ensembl (http://asia.ensemb).org/), and VEGA (http://vega.sanger.ac.uk/) database. Consequently, 8 genes are found in Sam gene family of the zebrafish and high homology of amino acid sequences between species is also identified (see FIGS. 1 and 2).

Further, it has been found that Sam gene family has the sequence, $CX_7CCX_{13}CXCX_{14}CX_{11}CX_4CX_5CX_{10}C$, which is a 10 regular cysteine structure, and this sequence is similar to that of a CC-type chemokine. To investigate a temporal and spatial expression pattern of the isolated Sam2 gene, whole-mount *in situ* hybridization is performed. As a result, it has been found that all Sam genes are expressed only in a central nervous system (see FIGS. 3 and 4), and cells expressing the Sam genes are neurons through the fact that Sam gene-expressing cells are increased in the *mind bomb* mutant (*mib*$^{la52b}$)[11] in which neurons are dramatically increased in the early of development (see FIG. 5).

In addition, it has been found that Sam2, among Sam genes, is expressed in a habenular nucleus from early development to the adult phase (see FIGS. 4 (c-g) and 6 (a-b)).

Further, to investigate a function of the Sam2 gene in development of a habenular nucleus and functional regulation, a Sam2 gene knockout (KO) zebrafish is constructed. Consequently, two types of mutants, in which bases of 5 *Sam2*$^{cnu1}$ and 17 *Sam2*$^{cnu2}$ are respectively deleted, such that the amino acid of Sam2 is not made, are constructed, and it has been found that the Sam2 knockout zebrafish does not show defects in survival, breeding and morphology (see FIG. 7).

Also, to investigate an influence of Sam2 gene loss in particular neuron development, Sam2 gene loss is compared in a Sam2 gene knockout zebrafish and control by using a dopamine neuron marker, i.e. *tyrosine hydroxylase (th), dopamine transporter (dat), nuclear receptor related 1 protein (nurr-1)*, a serotonin neuron marker (*tryptophan hydroxylase Raphe (tphR),* 5-hydroxytryptophan (5-HT)), an oxytocin neuron maker (*oxytosin(oxt)*) and an interpenduncular nucleus maker, i.e., *somatostatin1.1* (*sst1.1*). Consequently, it has been found that Sam2 gene loss of the Sam2 gene knockout zebrafish is not different from that of the control (see FIG. 8).

In addition, to investigate an expression pattern of a gene associated with a neurohormone of a Sam2 gene knockout zebrafish by using various molecular markers, expression patterns of genes associated with a neurohormone are compared in the Sam2 gene knockout zebrafish and control. As a result, it has been found that the expression pattern of the Sam2 gene knockout zebrafish is similar to that of the control (see FIG. 8 (g-l')

Further, a hypothalamus locus coeruleus projection is observed by using a *Tg(hcrt:mEGFP*) transformed Sam2 mutant fertilized egg. Consequently, it has been found that the hypothalamus locus coeruleus projection is normal by using the *Tg(hcrt.mEGFP*) transformed Sam2 mutant fertilized egg (see FIG. 8 (m-n').

Moreover, efferent axon development is observed in a Et(-1otpa:mmGFP)hd1 transformed Sam2 knockout zebrafish. Consequently, it has been found that axon development extended from a habenular nucleus to an interpenduncular nucleus is normally shown in the Et*(-1otpa:mmGFP)hd1* transformed Sam2 knockout zebrafish (see FIG. 6 (c-f)). Therefore, it has been found that the Sam2 gene does not directly affect development and movement of neurons and growth of axons.

In addition, to investigate an influence of Sam2 gene loss in a zebrafish on fear and anxiety, a Sam2 gene knockout zebrafish is placed on a novel tank and the ability to adapt to a new place is investigated. Consequently, it has been found that, although a locomotor activity such as a moving distance (see FIG. 9a) and average speed (see FIG. 9b) of a single individual of the Sam2 gene knockout zebrafish does not significantly differ from that of the control, anxiety-related behaviors (such as an abnormal behavior of bumping the head into the bottom of the tank (see FIG. 9c) and freezing bouts (see FIG. 9d)) in the Sam2 gene knockout zebrafish is significantly increased when compared to those of the control.

Further, from the thigmotaxis test result, it has been found that the Sam2 gene knockout zebrafish prefers the corner to the center (see FIG. 10). From the results, it has been found that the anxiety symptom of the Sam2 gene knockout zebrafish is observed higher than that of the control. Also, it has been found that, from the scototaxis test result, the anxiety degree of the Sam2 gene knockout zebrafish is higher than that of the control (see FIGS. 9 and 10).

Additionally, to investigate an influence of Sam2 gene loss in a zebrafish on fear and anxiety-associated behaviors, fear and anxiety behaviors of vertebrates are analyzed through a social behavior by using a Sam2 gene knockout zebrafish. Consequently, anxiety behaviors are observed in both the Sam2 gene knockout zebrafish and control during the pre adaptation phase. However, it has been found that, after adaptation, the control adapt to the environment and free swimming is observed, while the Sam2 gene knockout zebrafish does not adapt to the environment and shows continuous anxiety responses (see FIG. 11).

In addition, an anxiety response in group behaviors is measured through observation of social cohesion of a Sam2 gene knockout zebrafish. As a result, it has been found that, for the control, an inter-individual gap between fishes in the group after adaptation is increased than that of before adaptation, while an inter-individual gap between fishes of the Sam2 gene knockout zebrafish does not changed (see FIGS, 11 and 12).

Further, to investigate whether that the anxiety response observed in the Sam2 gene knockout zebrafish is caused by a neurotransmitter, i.e., serotonin system or stress-associated response, analysis is performed by using respective molecular markers. Consequently, it has been found that no difference is observed between anxiety responses of the control and Sam2 gene knockout zebrafish (see FIG. 13). Accordingly, it has been found that the Sam2 gene is involved in fear, anxiety and behavior control in the group. To investigate an influence of the Sam2 gene on behavior control by a habenular nucleus as a neurotransmitter, immunostaining is performed to investigate an influence of a human Sam2 gene on excitatory and inhibitory synapses through culture of rat hippocampal neurons. After overexpression of the Sam2 gene, vesicular GABA transporter (vGAT), gephyrin, and $GABA_A$ receptor a2 subunit ($GABA_AR$ a2), which are inhibitory synapse markers, are used for investigation. As a result, it has been found that the number and expression level of the inhibitory synapses are significantly reduced relative to the control.

It has been also found that the amplitude and frequency of the miniature inhibitory postsynaptic current (mIPSC) are significantly reduced when the Sam2 gene is overexpressed. In particular, it has been found that the influence of the Sam2 gene on inhibitory synapses affects neighbor neurons as well as the Sam2-treated nerves. Through the results, it has been found that the Sam2 gene is a secretory protein (see FIG. 14).

Further, to investigate expression of an excitatory neuromolecular marker due to Sam2 gene overexpression, immunostaining is performed. Consequently, it has been found that the Sam2 gene acts as a neuromodulator and enhances a nerve system activity such as weakening of a presynapse inhibitory function and enhancement of excitatory synapse (see FIG. 15).

In addition, patients are recruited to investigate autism and a hyperanxiety symptom due to Sam2 gene loss in humans, and autism and the hyperanxiety symptom due to Sam2 gene loss caused by microdeletion are investigated in the recruited patients. Consequently, by identifying autism and the hyperanxiety symptom due to Sam2 gene loss caused by microdeletion in the human, it has been found that the Sam2 gene is a key factor to regulate the anxiety response in the human (see FIG. 16).

Consequently, novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the novel samdori2 gene can be usefully applied to development of a pharmaceutical composition for preventing and treating relating mental diseases, in particular autism and depression.

The present invention also provides an expression vector including the samdori2 gene.

The samdori2 gene preferably includes any one of base sequences of SEQ ID NOS: 1-3 and 15-17, but not limited thereto.

Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the expression vector including the novel samdori2 gene can be useful.

Further, the present disclosure provides a transformant obtained by transforming a host cell with the expression vector.

The present disclosure also provides a method for preparing a recombinant transformant expressing samdori2, including:

1) preparing the expression vector including the samdori2 gene; and
2) transforming a host cell with the expression vector of step 1) according to the present invention.

A gene of step 1) preferably has any one of base sequences of SEQ ID NOS: 1-3 and 15-17, but not limited thereto.

Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the transformant, in which the expression vector including the novel samdori2 gene is transformed, can be useful.

Further, the present disclosure provides a samdori2 protein encoded by the samdori2 gene.

The present disclosure also provides a samdori2 protein having any one of amino acid sequences of SEQ ID NOS. 4-6 and 18-20.

Further, the present invention provides a pharmaceutical composition for preventing and treating a mental disease, the composition containing an expression vector including the samdori2 gene or samori2 protein.

The mental disease is preferably anxiety syndrome, depression, autism, manic-depressive illness, schizophrenia, mood disorders, sleep disorders, and attention deficit hyperactivity disorder (ADHD), but not limited thereto.

Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the novel samdori2 gene can be usefully applied as a pharmaceutical composition for preventing and treating relating mental diseases, in particular depression and autism.

The composition containing the Sam2 gene of the present invention may contain one or more active ingredients showing the same or similar function in addition to the ingredient described above.

The composition of the present invention may further include a pharmaceutically acceptable additive. As the pharmaceutically acceptable additive, starch, gelatinized starch, microcrystalline cellulose, milk sugar, povidone, colloidal silicon dioxide, calcium hydrogen phosphate, lactose, mannitol, crude maltose, Arabia gum, pregelatinized starch, corn starch, cellulose powder, hydroxypropyl cellulose, opadry, sodium starch glycolate, carnauba wax, synthetic aluminum silicate, stearic acid, magnesium stearate, aluminum stearate, calcium stearate, white sugar, dextrose, sorbitol, talc and so forth may be used. 0.1-90 parts by weight of the pharmaceutically acceptable additive according to the present invention is preferably included with respect to the composition, but not limited thereto.

In other world, the composition of the present invention may be administered in various oral and parenteral formulations for actual clinical administration. For formulation, preparation may be performed by using a normally used diluents or excipients such as a filler, an extender, a binder, a wetting agent, a disintegrating agent, and a surfactant. Examples of solid formulations for oral administration include a tablet, a pill, power, a granule, a capsule, etc. The solid formulations may be prepared by mixing a *Common carpesium* extract with at least one excipient such as starch, calcium carbonate, sucrose, lactose or gelatin. Further, a lubricant such as magnesium stearate, and talc may be used in addition to the simple excipient. Examples of liquid formulations for oral administration include a suspension, liquid for internal use, an emulsion, and a syrup. In addition to typically used diluents such as water, and liquid paraffin, various excipients such as a wetting agent, a sweetening agent, a flavoring agent, and a preservant may be included. Examples of preparations for parenteral administration may include a sterilized solution, a nonaqueous solvent, a suspension, an emulsion, a lyphophilized preparation, and suppository. As the nonaqueous solvent and suspending solvent, propylene glycol, polyethylene glycol, vegetable oil such as olive oil, injectable ester such as ethyl oleate may be used. As a substrate for the suppository, witepsol, macrogol, tween 61, cacao oil, laurinum, glycerogelati, etc. may be used.

The composition of the present invention may be orally administered or parenterally administered according to the desired method. For parenteral administration, topical skin, intraperitoneal injection, intrarectal injection, subcutaneous injection, intravenous injection, intramuscular injection or intrathoracic injection methods are preferably used. The dose varies depending on weight, age, sex, health status, and diet of patients, administration time, administration methods, excretion rates, and severity of a disease.

The composition according to the present invention is administered in a pharmaceutically effective amount. In the present invention, "a pharmaceutically effective amount" refers to an amount sufficient to treat a disease with a reasonable benefit/risk ratio available to apply a medicinal treatment. The level of effective dose may be determined depending on factors including types of diseases of a patient, severity, activity of a drug, sensitivity to a drug, administration time, administration routes, excretion rates, period of treatment, and a simultaneously used drug, and other factors well known in the medicinal field. The composition of the present invention may be administered as a separate therapeutic agent or administered in combination with other therapeutic agents. Also, the composition of the present invention may be sequentially or simultaneously added with the typical therapeutic agent, and the composition may be for single or multiple administrations. It is important to administer at a minimal dose which may lead a maximum effect without side effects in consideration of all of the factors described above, and the does may be easily determined by a person skilled in the art.

Specifically, an effective amount of the compound according to the present invention may vary depending on age, sex, and weight of a patient. Generally, 0.1 mg to 100 mg, and preferably, 0.5 mg to 10 mg per 1 kg of body weight may be administered daily or every other day, or administered 1 to 3 times per day. However, the dose may be increased or decreased depending on administration routes, severity of obesity, sex, weight, age, and so forth, so that the above dose the does not limit the scope of the present invention in any ways.

The present disclosure also provides a health food for preventing and alleviating a mental disease, the food containing an expression vector including the samdori2 gene samori2 protein.

The mental disease is preferably anxiety syndrome, depression, autism, manic-depressive illness, schizophrenia, mood disorders, sleep disorders, and attention deficit hyperactivity disorder (ADHD), but not limited thereto.

Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the samdori2 gene can be usefully applied as a health food for preventing and alleviating relating mental diseases, in particular depression and autism.

Types of the foods are not specifically limited. Examples of foods into which the above-described material may be added include various foods such as snacks, bread, and noodles, drink such as water, soft drink, and fruit beverage, gum, tea, vitamin complex, seasoning, health functional foods, and include all health functional foods in the typical meaning.

The samdori2 gene of the present disclosure may be added to foods as it is or used with other foods or food ingredients. Also, the samdori2 gene of the present invention may be appropriately used according to the typical method. The amount of a mixed active ingredient may be suitably determined depending on the purpose of use (for prevention or alleviation). Generally, the compound may be added to the health food in an amount of 0.01 to 15 wt% and preferably 0.1 to 5 wt% with respect to the total weight of the food. For health drink composition, the compound may be added in a ratio of 0.01 to 5.0 g, and preferably 0.01 to 1.0 g based on 100. However, for long term administration for the purpose of health control, the dose may be under the above-described range. Moreover, the active ingredient may be used in an amount above the range described above, because there is no problem in terms of safety.

The health functional drink composition of the present disclosure does not have specific limitation except the above described compound is included at the specified ratio as an essential ingredient, and includes various flavoring agent or natural carbohydrates may be included as additive ingredients as typical drinks. Examples of natural carbohydrates as described above include typical sugars such as monosaccharides, e.g., glucose, and fructose, disaccharides, e.g., maltose, and sucrose, and polysaccharides such as dextrin, and cyclodextrin; and sugar alcohol such as xylitol, sorbitol, and erythritol. In addition to those described above, natural flavoring agents (e.g., taumakin, and stevia extract) and synthetic flavoring agents (saccharin and aspartame) may be advantageously used. The ratio of the natural carbohydrates is about 0.1 to 2.0 g, and preferably about 0.1 to 1.0 g per 100 g of the composition of the present disclosure.

The health functional food of the present disclosure may be easily obtained by adding a process of adding the above described extract or compound of the present invention during the process of preparing a food used as a substrate, or adding a process of adding the above described extract or compound of the present invention after the preparation of a food used as a substrate. Taste and odor adjusting agents may be added.

In addition to those described above, the samdori2 gene of the present disclosure may contain various nutrients, vitamins, minerals (electrolytes), flavoring agents such as synthetic flavoring agents and natural flavoring agents, coloring agents and bulking agent (chess and chocolate), pectic acid and a salt thereof, alginic acid and a salt thereof, organic acids, protective colloid viscosity increasing agents, pH adjusting agents, stabilizing agents, preservants, glycerin, alcohol, carbonating agents used in soft drink. In addition to those described above, the samdori2 gene of the present invention may contain fleshes for preparation of natural fruit juice, fruit juice drink, and vegetable drink. These ingredients may be used alone or in combination with other ingredients. Although the ratio of the additive is not important, the samdori2 gene of the present invention is generally selected from the range of about 20 parts by weight per 100 parts by weight.

Further, the present disclosure provides a kit for diagnosing a mental disease, the kit including an antisense nucleotide for the samdori2 gene, a primer set or probe, or an aptamer or antibody for the samdori2 protein.

Novel tank, scototax is and social cohesion tests are performed on the novel Sam2 gene of the present disclosure by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the novel Sam2 gene of the present disclosure kit may be usefully applied to a kit for diagnosing a mental disease.

The present disclosure also provides a method for measuring an expression level of the samdori2 gene for providing information for diagnosis or monitoring a mental disease, the method including:

1) preparing cells isolated from a subject-derived habenular nucleus;
2) measuring an expression level of the samdori2 gene in the cells of step 1) according to the present invention; and
3) determining that a mental disease occurs or a risk for a mental disease is increased, when the samdori2 gene of step 2) according to the present invention is deleted or decreased when compared to that of a normal control.

The expression level in step 2) is preferably measured through any one selected from the group consisting of RT-PCR, ELISA, westem-blot, and immunohistochemistry, but not limited thereto.

Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the novel Sam2 gene of the present invention may be usefully applied to the method for measuring an expression level of samdori2 gene for providing information for diagnosis or monitoring a mental disease.

Further, the present disclosure provides a method for screening an agent for preventing or treating a mental disease, the method including:

1) treating cells expressing the samdori2 gene having any one of base sequences of SEQ ID NOS: 1-3, and 15-17, or the samdori2 protein encoded by the samdori2 gene with a subject;
2) measuring activity or expression of the samdori2 gene or samdori2 protein in step 1) according to the present invention; and
3) screening a subject sample which increases activity or expression of the samdori2 gene or samdori2 protein in step 2) according to the present disclosure.

Novel tank, scototaxis and social cohesion tests are performed on the novel Sam2 gene of the present disclosure by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the novel Sam2 gene of the present invention may be usefully applied to the method for screening an agent for preventing or treating mental disease.
The present disclosure also provides a model animal for a mental disease in which the samdori2 gene having any one of base sequences of SEQ ID NOS: 1-3 is knocked out in a habenular nucleus of an animal other than the human.
The animal is preferably a mouse, a rat, and a zebrafish, but not limited thereto.
The mental disease is preferably anxiety syndrome, depression, autism, manic-depressive illness, schizophrenia, mood disorders, sleep disorders, and attention deficit hyperactivity disorder (ADHD), but not limited thereto.
Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control and inhibitory neuron expression is decreased in neurons overexpressing the Sam2 gene. It has been also found that patients caused by Sam2 gene loss show autism and the hyperanxiety symptom. Therefore, the model animal in which the samdori2 gene having any one of base sequences of SEQ ID NOS: 1-3, and 15-17 is knocked out may be usefully applied.
Further, the present disclosure provides a method for preparing a model animal for a mental disease, including:

1) microinjecting a vector expressing a gene scissor specific for the samdori2 gene having any one of base sequences of SEQ ID NOS: 1-3, and 15-17 into a fertilized egg of an animal other than a human;
2) raising the animal in step 1) according to the present disclosure until the animal becomes an adult, and then breeding the animal with a wild type animal; and
3) screening an animal in which the samdori2 gene is knocked out among the bred animals in step 2) according to the present invention.

The mental disease is preferably anxiety syndrome, depression, autism, manic-depressive illness, schizophrenia, mood disorders, sleep disorders, and attention deficit hyperactivity disorder (ADHD), but not limited thereto.
Novel tank, scototaxis and social cohesion tests are performed on the novel Sam2 gene of the present invention by using the Sam2 gene-specific knockout zebrafish. It has been found that the zebrafish shows significantly higher fear and anxiety behaviors compared to the control, and inhibitory neuron expression is decreased in neurons overexpressing the Sam2 gene. It has been also found that patients caused by Sam2 gene loss show autism and the hyperanxiety symptom. Therefore, the method for preparing a model animal for a mental disease, in which the samdori2 gene having any one of base sequences of SEQ ID NOS: 1-3, and 15-17 is knocked out, may be usefully applied.
Hereinafter, the present disclosure will be described in more detail with reference to examples.
However, the following examples are provided to only illustrate the present invention.

## <EXAMPLE 1> RAISING OF ZEBRAFISH AND PREPARATION OF DEVELOPED EMBRYO

Wilde type, *mind bomb* mutant (mibta52b) and *Et(-1otpa::mmGFP)hd1* transformed zebrafishes were purchased from an aquarium (Seoul aquarium, Dajeon). The mind bomb (mibta52b) was a mutant obtained through ENU mutagenesis. Mind bomb heterozygote fishes, which is an individual having point mutation in which a RING domain are substituted to M1013R, were inbred and used for the experiment (Itoh, *et al*., 2003., National Institutes of Health (NIH), USA). The *Et(-1otpa::mmGFP)hd1* zebrafish was a novel enhancer trap zebrafish, and provided from Matthias Carl (Germany) and used. The prepared zebrafishes are raised under the following condition: temperature: 28.5°C, light and darkness: light on at 9:00 am to 10:00 pm, and light out other times. Fertilized eggs obtained from the zebrafishes were washed by using egg water (Sea Salts, 280 g), and allowed to develop on petri dishes. Then, the development processes were observed through a dissecting microscope. Developed embryos were selected according to time point and morphological changes, fixed with 4% paraformaldehyde/PBSw.

## <EXAMPLE 2> INVESTIGATION OF SAM2 GENE EXPRESSION IN HABENULAR NUCLEUS

### <2-1> ISOLATION OF SAMDORI GENE FROM ZEBRAFISH

To isolate the samdori gene from wild type, *mind bomb* mutant and Et(-1otpa::mmGFP)hd1 transformed zebrafishes, which were raised through the method as described in <Example 1>, reverse transcription polymerase chain reaction (RT-PCR) was used.

Specifically, mRNA was isolated by using the wild type raised through the method as described in <Example 1>. Complementary DNA was synthesized from 2 ug of the isolated mRNA by using RT transcript (Enzynomics, Korea). PCR was performed by using the Samdori primer which was synthesized from the complementary DNA and listed in Table 1 below (Table 1). Additionally, the GAPDH primer (SEQ ID NOS: 9 and 10) was used as a control gene (Table 1). After PCR has been completed, the sample was subjected to electrophoresis using 1.0% agarose gel including ethidium bromide, and then UV was irradiated to identify a band. The Sam2 band of 694 bp was cut to isolate DNA from the agarose (Elpis, Korea). The isolated Sam2 DNA was cloned into a T-vector (Promega, USA), and then a plasmid was isolated and purified to analyze the cloned Sam2 DNA gene by using miniprep DNA purification system kit (Intron, Korea). Then, the resultant was analyzed through an automated DNA sequencer.

[Table 1]

| SEQ ID NO. | Sequence |
|---|---|
| *sam2*, forward (SEQ ID NO 7) | 5'-ACGCCGCTGAATGAACCGATTACCGG-3' |
| *sam2*, reverse (SEQ ID NO 8) | 5'-GAGCGAACGCACTGCTTTACACAC-3' |
| *beta-actin,* forward (SEQ ID NO 9) | 5-GAGGAGCACCCCGTCCTGCTCAC-3' |
| *beta-actin*, reverse (SEQ ID NO 10) | 5-GATGGCTGGAACAGGGCCTCTGG-3' |

### <2-2> COMPARISON OF SIMILARITY ON CHROMOSOME

To investigate evolutional relationship between genomes of the human and zebrafish, the following method was conducted.

Specifically, to investigate evolutional relationship of a genome of a human and a gene isolated from a zebrafish through the method as described in Example <2-1>, amino acid sequences of the Sam2 gene of the human and the Sam2 gene of the zebrafish were searched by using Synteny database (http://teleost.cs.uoregon.edu/acos/synteny_db/), NCBI (http://www.ncbi.nlm.nih.gov/), Ensembl (http://asia.ensembl.org/), and VEGA (http://vega.sanger.ac.uk/) database, and then locations on the chromosomes were compared to investigate evolutional relationship on chromosomes.

Consequently, as shown in FIGS. 1 and 2, 8 genes were found in Sam gene family of the zebrafish, and high homology of amino acid sequences between species was found (FIGS. 1 and 2). It has been also found that the Sam gene family has 10 regular cysteine structure, $CX_7CCX_{13}CXCX_{14}CX_{11}CX_4CX_5CX_{10}C$ sequence, and these sequence has a similar form as CC-type chemokine.

### <2-3> INVESTIGATION OF TIME AND SPATIAL EXPRESSION PATTERN OF SAM2 GENE

To investigate a time and spatial expression pattern of the Sam2 gene, whole-mount *in situ* hybridization was performed.

Specifically, to investigate a time and spatial expression pattern of the Sam2 gene, agrp, cxcr4b, dat, hcrt, lov, mch, npy, otx5, oxt (itnp), pet-1, sst1,th, tphR, and Sam genes were cut with a restriction enzyme, and an anti-sense RNA probe was synthesized by using SP6, T7 or T3 polymerase (Fermenters, USA) and Digoxigenin-labelled UTP (Roche, Germany). Through an optical microscope, after fertilization, an appropriate phase of a fertilized egg at each step was added to 4% paraformaldehyde/PBT and fixed at 4°C for 12 hours or more. At least 20 hours after fertilization, a chorion of the fertilized egg was removed with tweezers and fixed such that a tail does not bend. For the fertilized egg before 20 hours of fertilization, a chorion was removed after fixing. In addition, from a tail bud phase (10 hours after fertilization), 0.2 mM phenylthiourea/embryonic water (PTU) was added to inhibit skin pigment formation. The fertilized egg, which was fixed after removal of chorion, was washed three times for 5 minutes with 1 X PBTw (phosphate buffered saline, 0.1% tween-20), and then replacement was performed about 3 to 4 times by using 100% methanol (MeOH). Then, the resultant was stored at a methanol state at -20°C. The developed embryo stored in methanol was washed in a stepwise manner for 5 minutes by using 75%, 50%, and 25% MeOH/PBSw. Then, washing was performed five times by using PBTw at a 5 minute interval. According to the developmental stage, the developed embryo was treated with PBTw including 10 ug/ml proteinase K. Thereafter, washing was performed 3 to 4 times at a 5 minutes interval by using PBTw. Then, the resultant was fixed with 4% paraformaldehyde/PBT at room temperature for 30 minutes or more. Washing was gently performed five times with PBTw at a 5 minute interval such that the developed embryo does not get damaged. Then, 300 ul HYB (50% formamide, 5X SSC, 0.1% Tween-20) was added and the resultant was stayed for 15 minutes at 68°C. Thereafter, 300 ul of 50% HYB (50% formamide, 5X SSC, 5 mg/ml torula RNA, 50 ug/ml heparin, citric acid, pH6, and 0.1% Tween-20) was added, and the resultant was subjected to prehybridization at 68°C for 1 to 2 hours or more. Thereafter, about 30 to 100 ng of the probe was added for hybridization at 68°C for 12 hours. While maintaining a temperature of 68°C, the probe was removed, and washing was sequentially performed for every 15 minutes by using 100%, 75%, 50% and 25% of HYB*/0.2X SSCTw. At the same temperature, replacement with 2XSSCTw was performed and washing was performed for 20 minutes. Then, washing was performed five times for every 30 minutes in 0.2XSSCT to remove a probe which was not hybridized. Thereafter, replacement was sequentially performed by using 75%, 50% and 25% of 0.2X SSCT/PBTw at 5 minute interval, and washing was performed five times for every 5 minutes with PBTw. To investigate color developed and expression regions, 300 ul of 5% sheep serum/PBTw was added, and the resultant was stayed at room temperature for 1 hour, such that a region, where the probe does not bind, was bound to serum to block color development and expression during antibody reaction. Then, 300 ul of anti-DIG-AP Fab (150 u/200 $\mu\ell$; Roche, Germany) diluted to 1/4000 in 5% sheep serum/PBTw was added, and the reaction was performed at room temperature for 4 hours or at 4°C for 12 to 16 hours. Washing was performed with PBTw six times for 10 minutes, six times for 20 minutes, 8 times for 30 minutes. Thereafter, washing was performed three times for 5 minutes by using a staining buffer (0.1 M Tris-Cl pH 9.5, 0.1 M NaCl, 50 mM $MgCl_2$, 0.1% Tween-20). A stock of nitro blue tetrazolium/5-bromo 4-chloro 3-indolyl phosphate (NBT/BCIP), which was a chromogenic substrate, was added to a staining buffer in an amount of 4.5 ul/ml (stock: dimethylformamide including 50 mg/ml NBT) or 3.5 ul/ml (stock: dimethylformamide including 50 mg/ml BCIP). Then, reaction was performed at room temperature, while blocking the light, and the stained region was investigated through observation with a microscope. The stained fertilized egg was washed four times for 5 minutes by using a stop solution (1XPBS pH 5.5, 1 mM EDTA, 0.1% Tween-20). Then, washing was performed with 100% methanol for 10 minutes about 4 to 5 times for dehydration, and then the resultant was stored at -20°C. The imaging process was performed by using the MZ-16 microscope (Leica, USA) in a 75% glycerol or benzyl benzoate:benzyl alcohol(2:1) solution.

Consequently, as shown in FIGS. 3 and 4, it has been found that all sam genes are expressed only in a central nervous system (FIGS. 3 and 4).

Also, as shown in FIG. 5, it has been found that cells expressing the *sam* genes are nerouns through the fact that *sam* gene-expressing cells are dramatically increased in the *mind bomb* mutant ($mib^{ta52b}$) in which neurons are dramatically increased during the early phase of development (FIG. 5).

Further, as shown in FIGS. 4 (c-g) and 6 (a-b), it has been found that Sam2, among the sam genes, is consistently expressed in a habenular nucleus from the early development to the adult phase (FIGS. 4 (c-g) and 6 (a-b)).

## <EXAMPLE 3> INVESTIGATION OF INFLUENCE OF SAM2 GENE LOSS ON PARTICULAR NEURON DEVELOPMENT OF ZEBRAFISH

### <3-1> CONSTRUCTION OF SAM2 GENE KNOCKOUT ZEBRAFISH

To investigate the role of Sam2 in development of a habenular nucleus and regulation of a function, a Sam2 gene knockout zebrafish was constructed by using a gene scissor technology, ZFN.

Specifically, to construct zinc-finger nuclease (ZFN) vector, which is a Sam2-specific gene scissor, a third exon of Sam2 was targeted, and zinc fingers respectively corresponding to left ZFN (TGCTCCTGCTTC, SEQ ID NO: 13) and right ZFN (CAGGTGGCAGGA, SEQ ID NO: 14) were used for combination in a modular assembly method to construct the vector. The constructed vector was cut with *Pvu*II restriction enzyme, and ZFN mRNA was synthesized by using T7 RNA polymerase. 2 to 3 ng of the ZFN mRNA constructed through the method was microinjected into a fertilized egg of a zebrafish in one cell stage. The microinjected zebrafish (F0) was raised until the zebrafish became an adult, and then bred with a wild type zebrafish. In the bred zebrafishes, Sam2 gene knockout was screened by using *T7 endonuclease* 1 method. The genotype of the Sam2 gene knockout zebrafish was screened by using primers listed in Table 2 below.

Consequently, as shown in FIG. 7, two types of mutant, in which bases of 5 $Sam2^{cnu1}$ and 17 $Sam2^{cnu2}$ were respectively damaged such that the amino acid of Sam2 was not made, were obtained, and it has been found that the Sam2 knockout zebrafish does not have defect in survival, breeding and morphology (FIG. 7).

[Table 2]

| SEQ ID NO. | Sequence |
|---|---|
| *Sam2$^{cnu1}$* forward (SEQ ID NO: 11) | 5'-TCTACTGAGGAGTGGTGTGA-3' |
| *Sam2$^{cnu1}$* reverse (SEQ ID NO: 12) | 5'-GGTCAGTTTCAGAGAGCTGG-3' |
| left ZFN (SEQ ID NO: 13) | TGCTCCTGCTTC |
| right ZFN (SEQ ID NO: 14) | CAGGTGGCAGGA |

**<3-2> INVESTIGATION OF INFLUENCE OF SAM2 GENE LOSS ON PARTICULAR NEURON DEVELOPMENT**

To investigate influence of sam2 gene loss on particular neuron development by using the Sam2 gene knockout zebrafish prepared through the method as described in Example <3-1>, the following method was conducted.

Specifically, an influence of sam2 gene loss on particular neuron development was investigated by using a dopamine neuron marker, i.e. *tyrosine hydroxylase (th), dopamine transporter (dat),* and *nuclear receptor related 1 protein (nurr-1),* a serotonin neuron marker (*tryptophan hydroxylase Raphe (tphR),* 5-hydroxytryptophan (5-HT)), an oxytocin neuron marker *(oxytosin (oxt)*) and an interpenduncular nucleus marker, i.e., *somatostatin1.1* (*sst1.1*). *th* and *dat* DNA, which were expressed in dopamine neuron, were cut with a restriction enzyme. Then, *in vitro* transcription was performed by using RNA polymerase to prepare an mRNA probe. The prepared mRNA probe was used to perform whole-mount *in situ* hybridization.

Consequently, as shown in FIG. 8, it has been found that, when comparing Sam2 gene loss in the Sam2 gene knockout zebrafish and control, Sam2 gene loss of the Sam2 gene knockout zebrafish is not different from that of the control (FIG. 8).

**<3-2> INVESTIGATION OF EXPRESSION PATTERN OF GENE ASSOCIATED WITH NEUROHORMONE OF SAM2 GENE KNOCKOUT ZEBRAFISH**

To investigate an expression pattern of a gene associated with neurohormone of a Sam2 gene knockout zebrafish by using the Sam2 gene knockout zebrafish prepared through the method as described in Example <3-1>, the following method was conducted.

Specifically, DNA of *hypocretinlorexin (hcrt), melanin concentrating hormome* (*mch*), *agouti related protein homolog* (*agrp*), *neuropeptide Y* (*npy*), and *fev* (*ETS oncogene family;pet-1*) were cut with a restriction enzyme. Then, *in vitro* transcription was performed by using RNA polymerase to prepare an mRNA probe. The constructed mRNA probe was used to perform whole-mount *in situ* hybridization.

Consequently, as shown in FIG. 8 (g-l'), it has been found that, when expression patterns of the gene associated with neurohormone of the Sam2 gene knockout zebrafish and control, the expression pattern of the gene associated with neurohormone of the Sam2 gene knockout zebrafish is similar to that of the control (FIG. 8 (g-l')).

**<3-3> OBSERVATION OF HYPOTHALAMUS LOCUS COERULEUS PROJECTION BY USING Tg(hcrt:mEGFP) TRANSFORMED SAM2 MUTANT FERTILIZED EGG**

To observe hypothalamus locus coeruleus projection by using a *Tg(hcrt:mEGFP)* transformed Sam2 mutant fertilized egg, the following method was conducted.

Specifically, an F1 adult zebrafish of *Tg(hcrt:mEGFP*), which was a transformant expressing a fluorescent protein in a hypothalamus locus coeruleus projection, was bred with a homozygote adult zebrafish having sam2 gene loss. Then, from developed embryos of 48 hours obtained through breeding, an individual expressing fluorescence was selected by using a fluorescent microscope, and raised until the embryo became an adult. Through inbreeding of heterozygote zebrafishes having Sam2 gene loss and expressing fluorescence, a homozygote developed embryo having Sam2 gene loss and expressing fluorescence was obtained. Then, 83 hours after fertilization of homozygote developed embryo, a hypothalamus locus coeruleus projection was observed through a fluorescence microscope.

Consequently, it has been found that the hypothalamus locus coeruleus projection is normal by using *Tg(hcrt:mEGFP*) transformed Sam2 mutant fertilized egg (FIG 8 (m-n')).

### <3-4> OBSERVATION OF DEVELOPMENT OF EFFERENT AXON OF *Et(-1otpa:mmGFP)hd1* TRANSFORMED Sam2 KNOCKOUT ZEBRAFISH

To observe development of an efferent axon of *Et(-1otpa:mmGFP)hd1* transformed Sam2 knockout zebrafish, the following method was conducted.

Specifically, an adult zebrafish of *Et(-1otpa:mmGFP)hd1,* which was a transformant expressing a fluorescent protein in a nerve connected to a habenular nucleus, was bred with a homozygote adult zebrafish having sam2 gene loss. Then, from developed embryos of 48 hours obtained through the breeding, an individual expressing fluorescence was selected by using a fluorescent microscope, and raised until the embryo became an adult. Through inbreeding of heterozygote zebrafishes having Sam2 gene loss and expressing fluorescence, a homozygote developed embryo having Sam2 gene loss and expressing fluorescence was obtained. The homozygote developed embryo having Sam2 gene loss obtained through the method was fertilized, and, after 4.5 days of fertilization, a habenular nucleus nerve was observed through a fluorescence microscope.

Consequently, as shown in FIG. 6 (c-f), it has been found that development of an efferent axon extended from a habenular nucleus toward an interpenduncular nucleus was shown in normal in the *Et(-1otpa:mmGFP)hd1* transformed *Sam2* knockout zebrafish (FIG. 6 (c-f)).

Therefore, it has been found that the Sam2 gene does not directly affect on development and movement of neurons and growth of axons.

## <EXAMPLE 4> INVESTIGATION OF INFLUENCE OF SAM2 GENE LOSS ON FEAR AND ANXIETY OF ZEBRAFISH

To investigate an influence of Sam2 gene loss on fear and anxiety of a zebrafish, open tank, novel tank and dark/light preference (scototaxis) tests were performed on the Sam2 gene knockout zebrafish prepared through the method as described in Example <3-1>.

Specifically, to investigate a fear response, a male Sam2 gene knockout zebrafish prepared through the method as described in Example <3-1> was placed in a novel tank (15 cm > 15 cm >25 cm; height > length > width) for 10 minutes, and behaviors were observed through video recording. To perform fear response analysis through the scototaxis test, a zebrafish was placed in a box having white and black regions and allowed to adapt for 5 minutes. Then, behaviors were analyzed through video recording for 15 minutes. To compare the fear responses of the zebrafishes, a heterozygote adult zebrafish without Sam2 gene loss among descendants obtained by inbreeding of heterozygote adult zebrafishes having Sam2 gene loss was used as a control. For the behavior analysis, EthoVision XT7 was used, and statistical analysis was performed through Student's t-test.

Consequently, as shown in FIG. 9, it has been found that, as a result of the novel tank test to investigate an ability to adapt a novel place, a moving distance (FIG. 9a), an average speed (FIG. 9b) and a locomotor activity of a single individual of the Sam2 gene knockout zebrafish are not significantly different from those of the control, however, anxiety responses of the Sam2 gene knockout zebrafish such as an abnormal behavior of bumping a head into the bottom of the tank (FIG 9c) and freezing bouts (FIG. 9d) are significantly increased.

Further, as shown in FIGS. 9 and 10, it has been found that, trough the thigmotaxis test result, the *Sam2* gene knockout zebrafish prefers the corner to the center (FIG. 10). Through the result, it has been found that the anxiety symptom of the Sam2 gene knockout zebrafish is observed higher. Moreover, it has been found that the anxiety degree of the Sam2 gene knockout zebrafish is higher than that of the control through the scototaxis test (FIGS. 9 and 10).

## <EXAMPLE 5> INVESTIGATION OF SAM2 GENE LOSS ON FEAR AND ANXIETY OF ZEBRAFISH

### <5-1> MEASUREMENT OF FEAR AND ANXIETY THROUGH OBSERVATION OF BEHAVIOR INDEX OF SAM2 GENE KNOCKOUT ZEBRAFISH

To analyze fear and anxiety responses of vertebrates through social behaviors by using the Sam2 gene knockout zebrafish prepared through the method as described in Example <3-1>, the following method was conducted.

Specifically, It has been reported that formation of a group called shoaling, which is an innate response of fishes to cope with predators, is used as a behavior index to measure fear and anxiety (Gerlai. R, Behav Brain Res, 2010, 207, 223-231). Therefore, through the social behavior of the Sam2 gene knockout zebrafish in a group, fear and anxiety responses of vertebrates were analyzed. Through the method as described in <Example 4>, 5 Sam2 gene knockout zebrafishes and 5 controls were placed in a novel tank, and flow and locations of the zebrafishes in the group were recorded through a video for 30 minutes and analyzed (FIGS. 11 and 12). Behaviors for 5 to 8 minutes before habitation of the zebrafishes and behaviors for 13 to 16 minutes after habitation were analyzed at a 20 second interval, and an individual gap between fishes was calculated by using the following [Formula 1]. Moreover, statistical analysis was performed by using a SigmaPlot software.

Consequently, as shown in FIG. 11, it has been found that, both the Sam2 gene knockout zebrafish and control show the anxiety responses before adaptation, but after adaptation, the control adapt to the environment and free swimming was observed, while the Sam2 gene knockout zebrafish does not adapt to the environment and shows the continuous anxiety response (FIG. 11).

[Formula 1]

$$\text{Distance} = \sqrt{(x_a - x_0)^2 + (y_a - y_0)^2 + (z_a - z_0)^2}$$

Wherein, $x_0$, $y_0$ and $z_0$ indicate a fish located at the center in the fish group; and $x_a$, $y_a$ and $z_a$ indicate shoaling fishes.

### <5-2> MEASUREMENT OF ANXIETY RESPONSE IN GROUP BEHAVIOR THROUGH OBSERVATION OF SOCIAL COHESION OF SAM2 GENE KNOCKOUT ZEBRAFISH

To measure the anxiety response in group behaviors through observation of social cohesion of the Sam2 gene knockout zebrafish prepared through the method as described in Example <3-1 >, the following method was conducted.

Specifically, social cohesion which measures an inter-individual gap between fishes in a group is an index for the anxiety response in group behaviors. The anxiety response of the Sam2 gene knockout zebrafish was analyzed through the method as described in Example <5-1>, and an individual gap between fishes was calculated by using the above [Formula 1]. Moreover, statistical analysis was performed by using the SigmaPlot software.

Consequently, as shown in FIGS. 11 and 12, it has been found that, for the control, the inter-individual gap between fishes in the group after adaptation is increased than that of before adaptation, while the inter-individual gap between Sam2 gene knockout zebrafishes does not changed (FIGS. 11 and 12).

### <5-3> INVESTIGATION OF ANXIETY RESPONSE OF SAM2 GENE KNOCKOUT ZEBRAFISH BY USING MOLECULAR MARKER

To investigate whether the anxiety response observed in the Sam2 gene knockout zebrafish prepared through the method as described in Example <3-1> is caused by a neurotransmitter, serotonin system, or stress-related response, corresponding molecular markers were used.

Specifically, to isolate brains of one month and 3 months of heterozygote and homozygote having Sam2 gene loss, each fish was placed in a 15 ml tube, and fixed with 4% paraformaldehyde/PBTw. After 12 hours, the brain of the fish was isolated and dehydrated with methanol. Then, whole-mount *in situ* hybridization was performed. DNAs of *tphR, slc6a4a* and *pomca* were cut, and an mRNA probe was constructed through *in vitro* transcription by using RNA polymerase. Whole-mount *in situ* hybridization was performed by using the constructed mRNA probe.

Consequently, as shown in FIG. 13, no difference was observed in the anxiety responses of the control and Sam2 gene knockout zebrafish (FIG. 13).

Therefore, it has been found that the *Sam2* gene is associated with fear, anxiety and behavior control in the group through the result of Example <5-3>.

## <EXAMPLE 6> INVESTIGATION OF INFLUENCE OF SAM2 GENE ON BEHAVIOR CONTROL BY HABENULAR NUCLEUS AS NEUROTRANSMITTER

### <6-1> INVESTIGATION OF INFLUENCE OF HUMAN SAM2 GENE ON EXCITATORY AND INHIBITORY SYNAPSES THROUGH RAT HIPPOCAMPAL NEURON CULTURE

To investigate whether the Sam2 gene is associated with behavior control by a habenular nucleus as a neurotransmitter, immunostaining was performed.

Specifically, hippocampal neurons on Day 18 of BALB/c mouse embryo development were cultured in a B27 cell culture medium (Animal Experiment committee of Medical College of Wisconsin, IACUC). After 14 days of the *in vitro* culture, the Sam2 gene was introduced into the neuron by using lipofectamine 2000 reagent. Immunostaining and electrophysiological test were performed on neurons after 17 to 18 days of *in vitro* culture. The neurons were placed in a fixing solution including 2% formaldehyde, 4% sucrose and 1 × PBS for 2 minutes, and then pretreated with cold methanol for 10 minutes. After pretreatment, the sample was allowed to react with primary antibodies (rat anti-HA (Roche; 1:250), mouse anti-vGAT (Synaptic systems; 1:250), mouse anti-vGLUT (Millipore; 1:250), rabbit anti-GABAAR 2 (Synaptic systems; 1:500), mouse anti-PSD-95 (NeuroMab; 1:400), mouse anti-gephyrin (Synaptic systems; 1:1,000), mouse anti-Gal (Promega; 1:1,000), alc rabbit anti-Gal (Abcam; 1:5,000)) and secondary antibodies (Alexa Fluor488 (LifeTechnologies), Cy-3 and Cy5-conjugated (Jackson ImmunoResearch Laboratories)) for a day. Then, the electrophysiological test was performed by measuring miniature inhibitory postsynaptic current (mIPSC) through whole-cell patch recordings after Sam2 gene overexpression. In addition, mIPSC was analyzed by using the Mini Analysis software.

Consequently, as shown in FIG. 14, it has been found that, as a result of investigation using inhibitory synapse markers (vesicular GABA transporter (vGAT), gephyrin, and $GABA_A$ receptor a2 subunit ($GABA_AR$ a2)) after Sam2 gene overexpression, the number and expression level of the inhibitory synapses are significantly decreased than those of the control. In particular, it has been found that the influence of the Sam2 gene on the inhibitory synapse was equally applied to neighbor neurons in addition to the neuron treated with Sam2, and therefore the Sam2 gene is a secretory protein (FIG. 14).

### <6-2> INVESTIGATION OF EXCITATORY NEURON MARKER THROUGH SAM2 GENE OVEREXPRESSION

To investigate expression of an excitatory neuron marker through Sam2 gene overexpression, immunostaining was performed.

Specifically, to investigate expression of an excitatory neuron marker through Sam2 gene overexpression, excitatory neuron markers, i.e., vesicular glutamate transporter (vGLUT) and PSD-95 were analyzed. Neurons were fixed in a fixing solution including 2% formaldehyde, 4% sucrose and 1 x PBS for 2 minutes, and dehydrated for 10 minutes by using methanol stored at -20°C, Then, washing was sequentially performed by using 75%, 50%, and 25% methanol/PBS solution. Primary antibodies (for inhibitory synapse marker: mouse anti-vGAT (Synapse system, 1:250), rabbit anti-GABAARa2 (Synapse system, 1:500), mouse anti-p-Gal (Promega, 1:1,000); for excitatory synapse marker: mouse anti-PSD-95 (NeuroMab, 1:400), mouse anti-vGLUT (Millipore, 1:250) were added to the washed neurons, and reacted. The resultant was washed by using 1 X PBS, and then secondary antibodies (Alexa Fluor488 (LifeTechnologies), Cy3- and Cy5-conjugated (Jackson ImmunoResearch Laboratories)) were added and reacted. Thereafter, neurons stained with the inhibitory and excitatory synapse markers were observed under a fluorescent microscope.

Consequently, as shown in FIG. 15, it has been found that the Sam2 gene acts as a neurotransmitter to thereby increase the activity of the nervous system such as decline in presynapse inhibitory function and enhancement of excitatory synapse (FIG. 15).

## <EXAMPLE 7> INVESTIGATION OF AUTISM AND HYPERANXIETY SYMPTOM IN HUMAN DUE TO SAM2 GENE LOSS

### <7-1> RECRUIT OF PATIENTS

Copy number variation was investigated by performing array comparative genome hybridization (aCGH) to 35,000 patients.

### <7-2> INVESTIGATION OF AUTISM AND HYPERANXIETY SYMPTOM IN HUMAN DUE TO SAM2 GENE LOSS CAUSED BY MICRODELETION

To investigate autism and the hyperanxiety symptom due to Sam2 gene loss caused by microdeletion in patients recruited through the method as described in Example <7-1>, the following method was conducted.

Specifically, autism and the hyperanxiety symptom due to Sam2 gene loss were diagnosed through counseling with a doctor and patients recruited through the method as described in Example <7-1>.

Consequently, as shown in FIG. 16, it has been found that patients have autism and the hyperanxiety symptom (excessive fear) due to Sam2 gene loss caused by microdeletion are identified in the human and therefore the Sam2 gene is an important factor in the human to control the anxiety response (FIG. 16).

## Claims

1. A pharmaceutical composition comprising a samdori2 (sam2) protein consisting of amino acid sequence of SEQ ID NO: 5 as active ingredients for use in preventing or treating a mental disease.

2. The pharmaceutical composition for use in preventing or treating a mental disease of claim 1, wherein the mental disease is anxiety syndrome, depression, autism, manic-depressive illness, schizophrenia, mood disorders, sleep disorders, or attention deficit hyperactivity disorder (ADHD).

3. A model animal for a mental disease in which a samdori2 gene consisting of base sequence of SEQ ID NO: 2 is knocked out in an animal other than a human.

4. The model animal for a mental disease of claim 3, wherein the animal is a mouse, a rat, or a zebrafish.

5. The model animal for a mental disease of claim 3, wherein the mental disease is anxiety syndrome, depression, autism, manic-depressive illness, schizophrenia, mood disorders, sleep disorders, or attention deficit hyperactivity disorder (ADHD).

## Patentansprüche

1. Pharmazeutische Zusammensetzung, umfassend ein samdori2 (sam2)-Protein, bestehend aus der Aminosäuresequenz von SEQ ID NO: 5, als aktive Bestandteile zur Verwendung bei der Vorbeugung oder Behandlung einer Geisteskrankheit.

2. Die pharmazeutische Zusammensetzung zur Verwendung bei der Vorbeugung oder Behandlung einer Geisteskrankheit nach Anspruch 1, wobei die Geisteskrankheit Angstsyndrom, Depression, Autismus, manisch-depressive Krankheit, Schizophrenie, Stimmungsstörungen, Schlafstörungen oder Aufmerksamkeitsdefizit-Hyperaktivitätsstörung (ADHS) ist.

3. Modelltier für eine Geisteskrankheit, bei dem ein Samdori2-Gen, bestehend aus der Basensequenz von SEQ ID NO: 2, in einem anderen Tier als einem Menschen ausgeschaltet ist.

4. Das Modelltier für eine Geisteskrankheit nach Anspruch 3, wobei das Tier eine Maus, eine Ratte oder ein Zebrafisch ist.

5. Das Modelltier für eine Geisteskrankheit nach Anspruch 3, wobei die Geisteskrankheit Angstsyndrom, Depression, Autismus, manisch-depressive Krankheit, Schizophrenie, Stimmungsstörungen, Schlafstörungen oder Aufmerksamkeitsdefizit-Hyperaktivitätsstörung (ADHS) ist.

## Revendications

1. Composition pharmaceutique comprenant une protéine samdori2 (sam2) consistant en une séquence d'acides aminés SEQ ID NO :5 comme composant actif, pour utilisation dans la prévention ou le traitement d'une maladie mentale.

2. La composition pharmaceutique pour utilisation dans la prévention ou le traitement d'une maladie mentale selon la revendication 1, dans laquelle la maladie mentale est un syndrome d'anxiété, une dépression, un autisme, une psychose maniaco-dépressive, une schizophrénie, des troubles de l'humeur, des troubles du sommeil, ou un trouble de déficit de l'attention avec hyperactivité (TDAH).

3. Un modèle animal pour une maladie mentale dans lequel un gène samdori2 consistant en séquence de base SEQ ID NO :2 est inactivée dans un animal autre qu'un humain.

4. Le modèle animal pour une maladie mentale selon la revendication 3, dans lequel l'animal est une souris, un rat, ou un poisson zèbre.

5. Le modèle animal pour une maladie mentale selon la revendication 3, dans lequel la maladie mentale est un syndrome d'anxiété, une dépression, un autisme, une psychose maniaco-dépressive, une schizophrénie, des troubles de l'humeur, des troubles du sommeil, ou un trouble de déficit de l'attention avec hyperactivité (TDAH).

[Fig. 1]

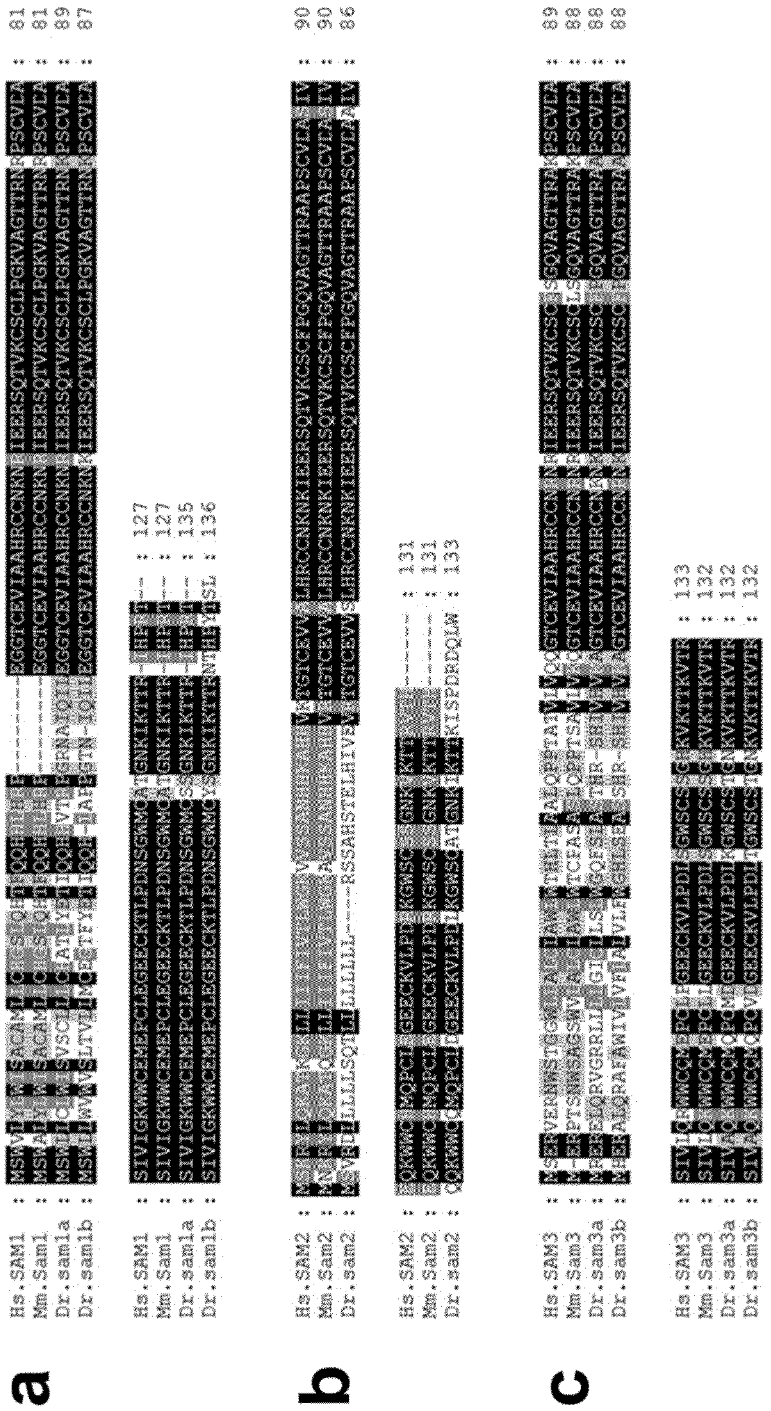

[Fig. 2]

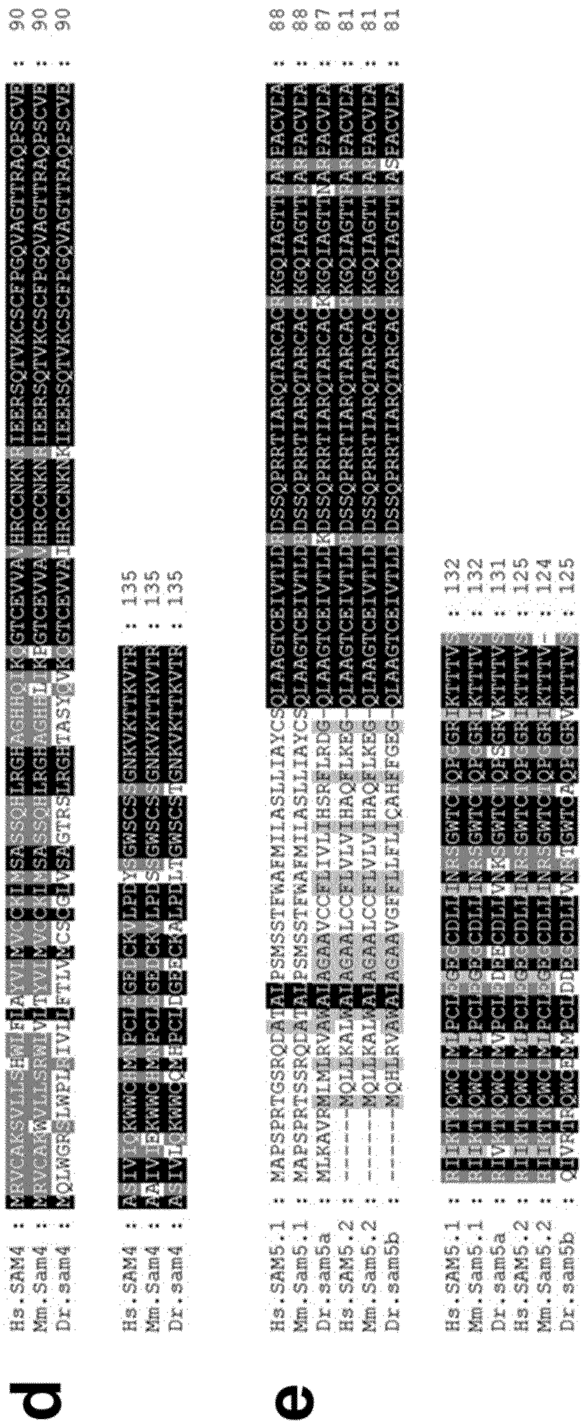
d
Hs.SAM4
Mm.Sam4
Dr.sam4
e
Hs.SAM5.1
Mm.Sam5.1
Dr.sam5a
Hs.SAM5.2
Mm.Sam5.2
Dr.sam5b

[Fig. 3]

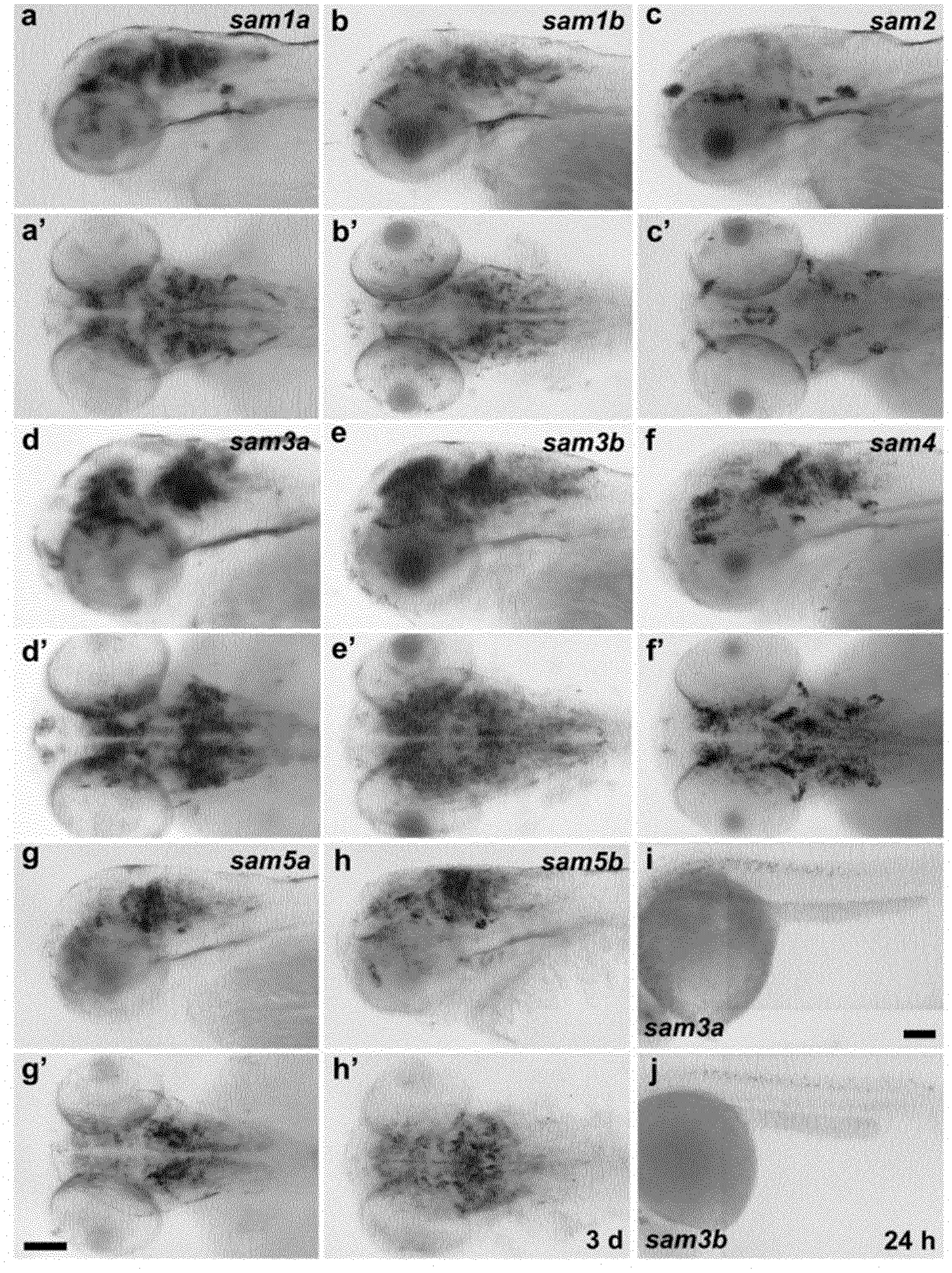
a
sam1a
b
sam1b
c
sam2
a'
b'
c'
d
sam3a
e
sam3b
f
sam4
d'
e'
f'
g
sam5a
h
sam5b
i
sam3a
g'
h'
3 d
j
sam3b
24 h

[Fig. 4]

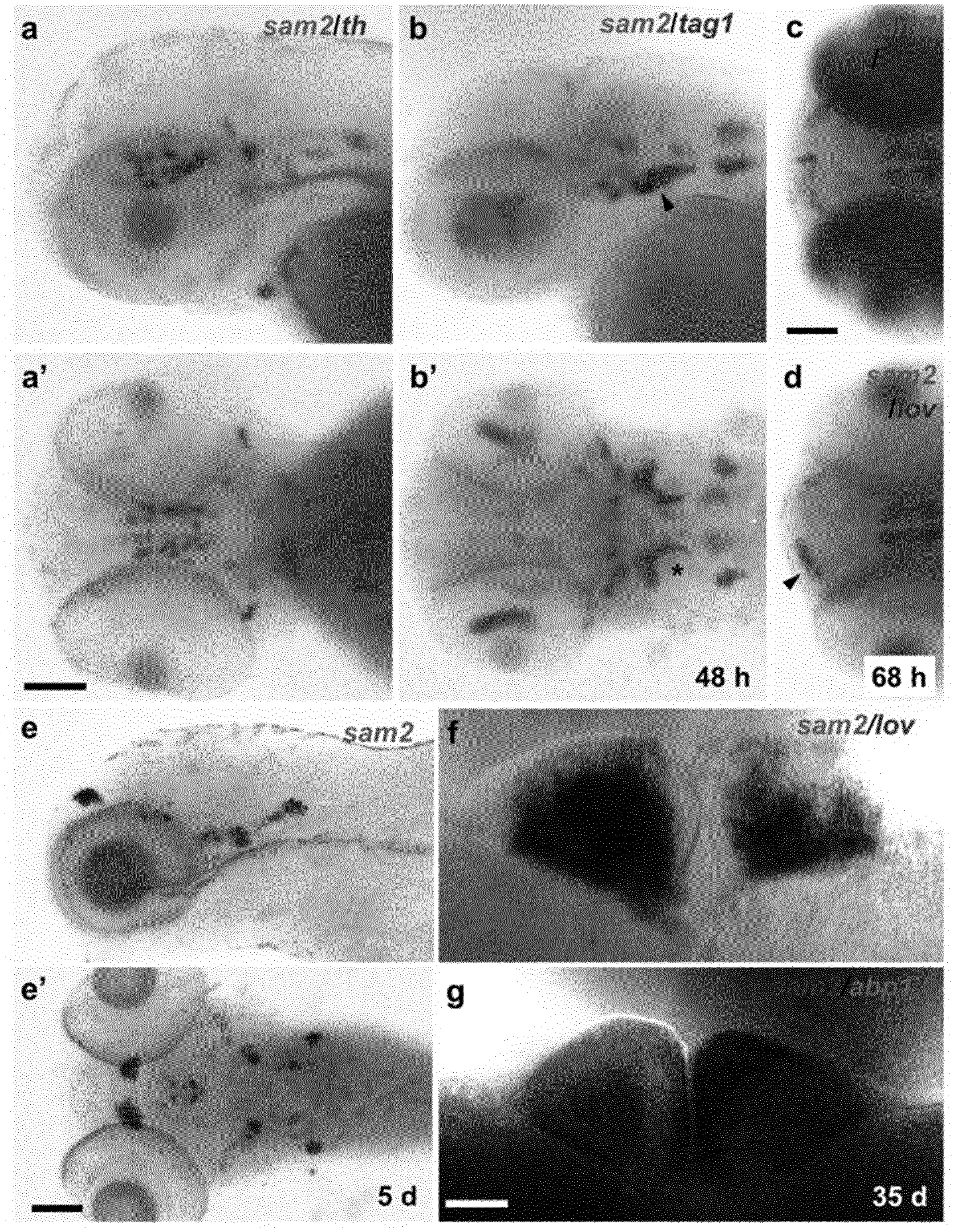
a
sam2/th
b
sam2/tag1
c
a'
b'
*
48 h
d
sam2
/lov
68 h
e
sam2
f
sam2/lov
e'
5 d
g
35 d

[Fig. 5]

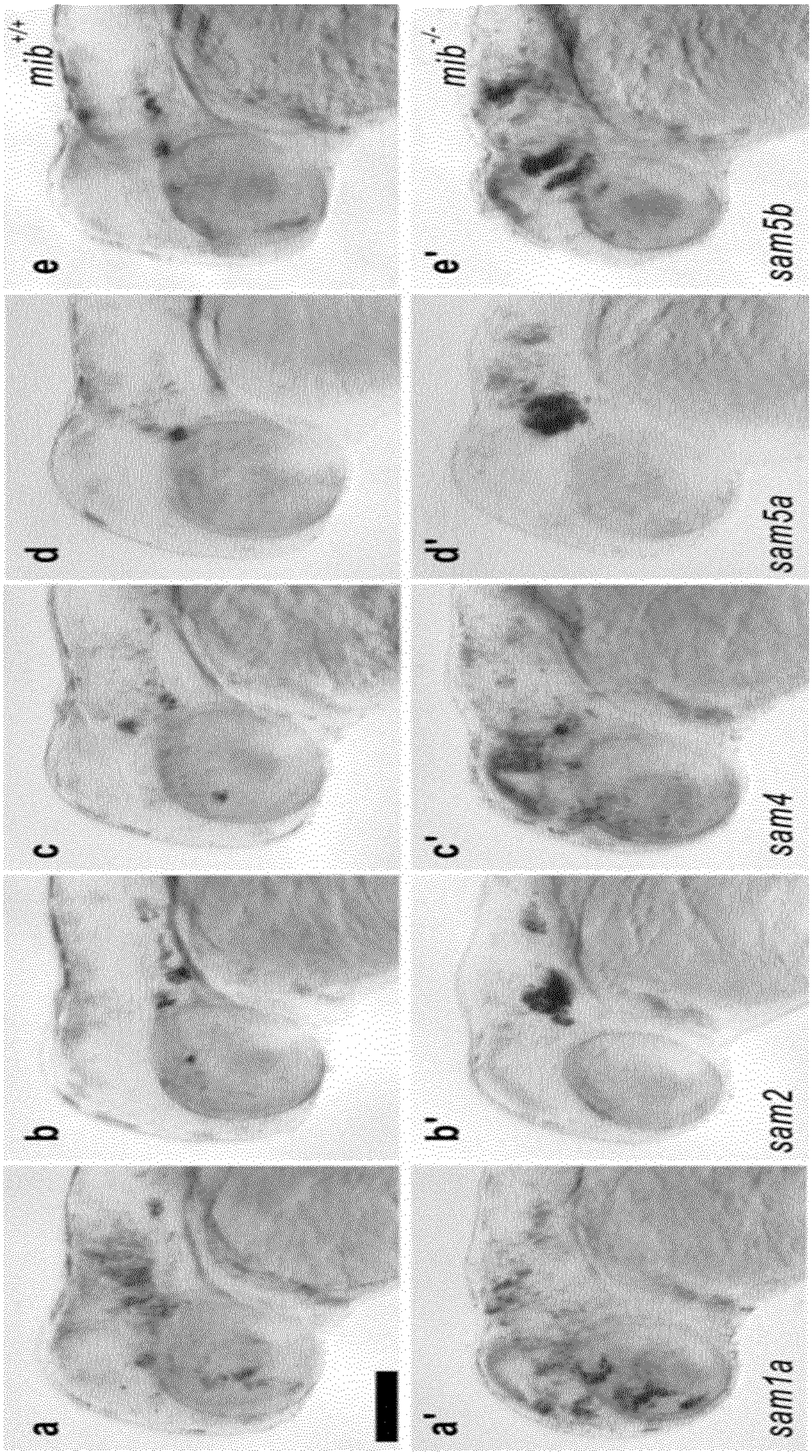

[Fig. 6]

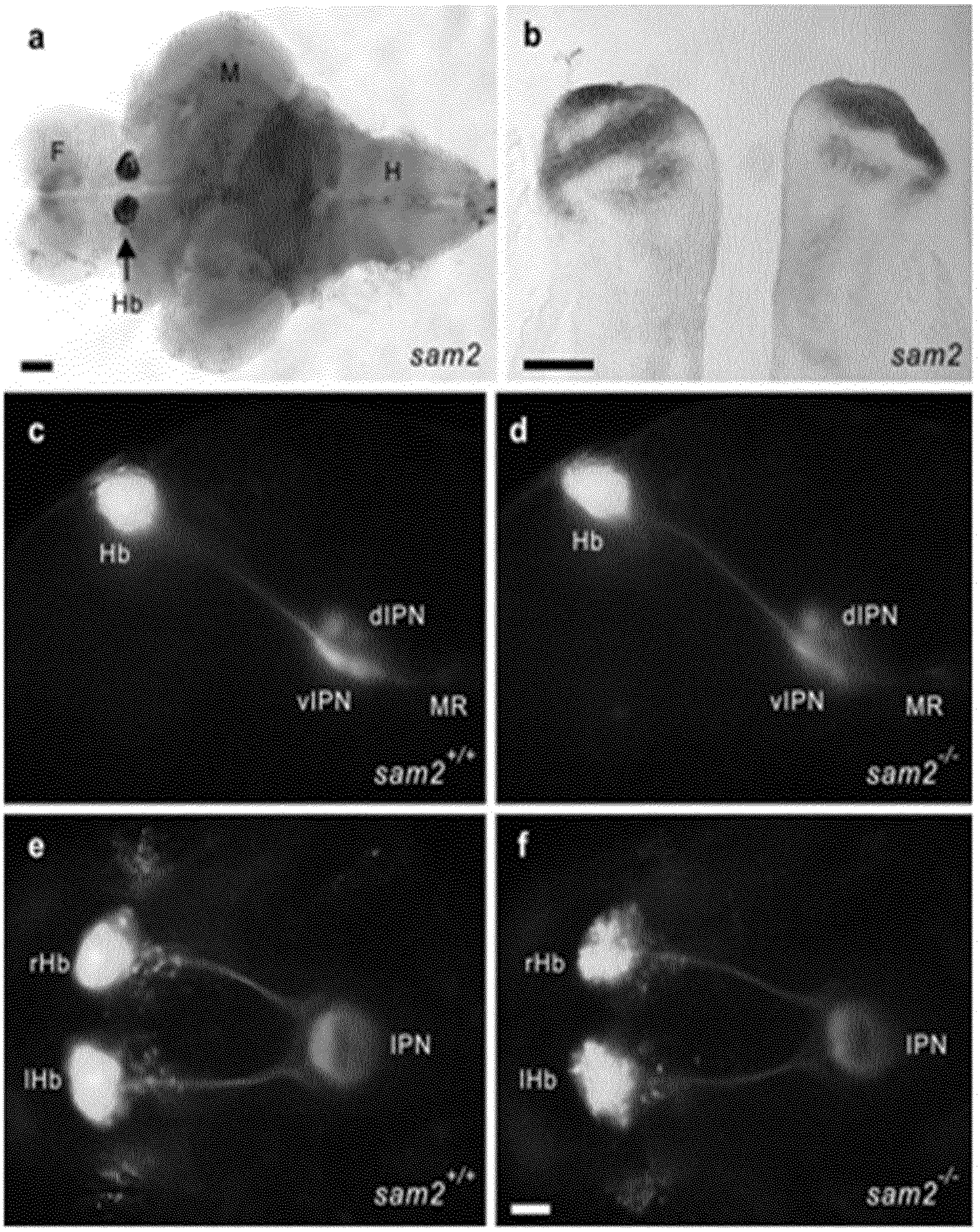
a
M
F
H
Hb
sam2
b
sam2
c
Hb
dIPN
vIPN
MR
sam2+/+
d
Hb
dIPN
vIPN
MR
sam2-/-
e
rHb
IPN
lHb
sam2+/+
f
rHb
IPN
lHb
sam2-/-

[Fig. 7]

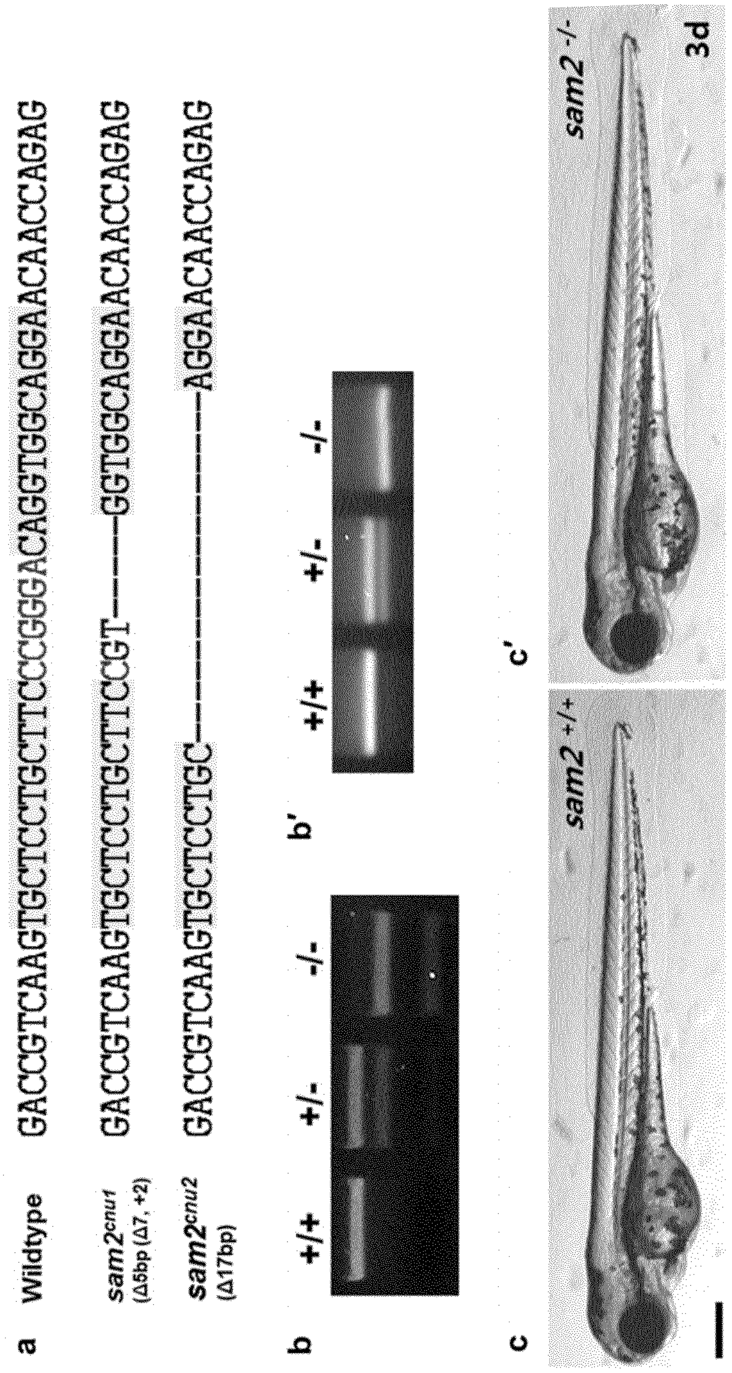
a
Wildtype
GACCGTCAAGTGCTCCTGCTTCCCGGGACAGGTGGCAGGAACAACCAGAG
sam2cnu1 (Δ5bp (Δ7, +2))
GACCGTCAAGTGCTCCTGCTTCCGT-----GGTGGCAGGAACAACCAGAG
sam2cnu2 (Δ17bp)
GACCGTCAAGTGCTCCTGC-----------------AGGAACAACCAGAG
b
+/+
+/-
-/-
b'
+/+
+/-
-/-
c
sam2 +/+
c'
sam2 -/-
3d

[Fig. 8]

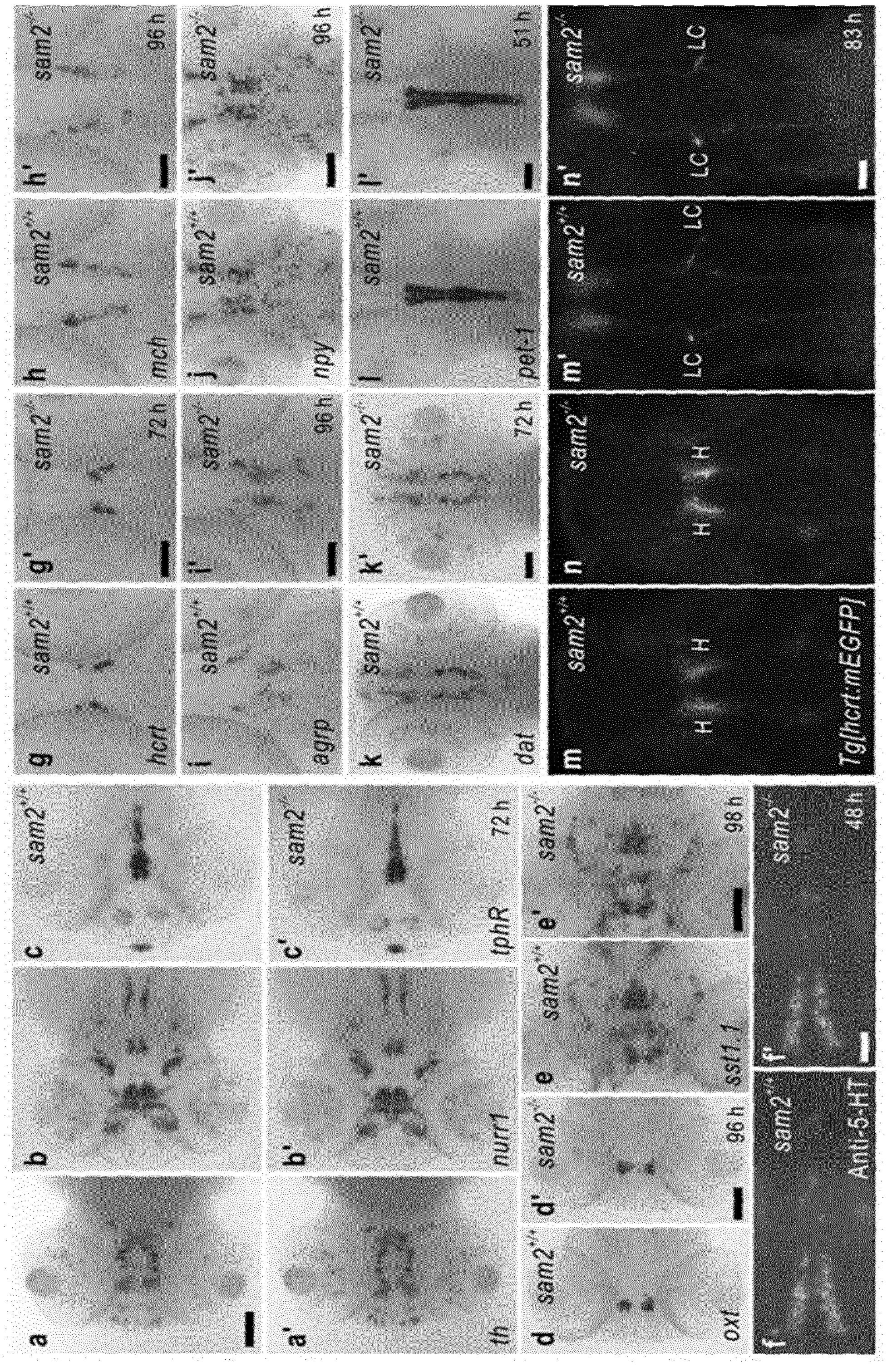
a
a'
th
b
b'
nurr1
c
c'
tphR
72 h
d
d'
oxt
96 h
e
e'
sst1.1
98 h
f
f'
Anti-5-HT
48 h
g
g'
hcrt
72 h
h
h'
mch
96 h
i
i'
agrp
96 h
j
j'
npy
96 h
k
k'
dat
72 h
l
l'
pet-1
51 h
m
n
Tg[hcrt:mEGFP]
H
m'
n'
LC
83 h
sam2+/+
sam2-/-

[Fig. 9]

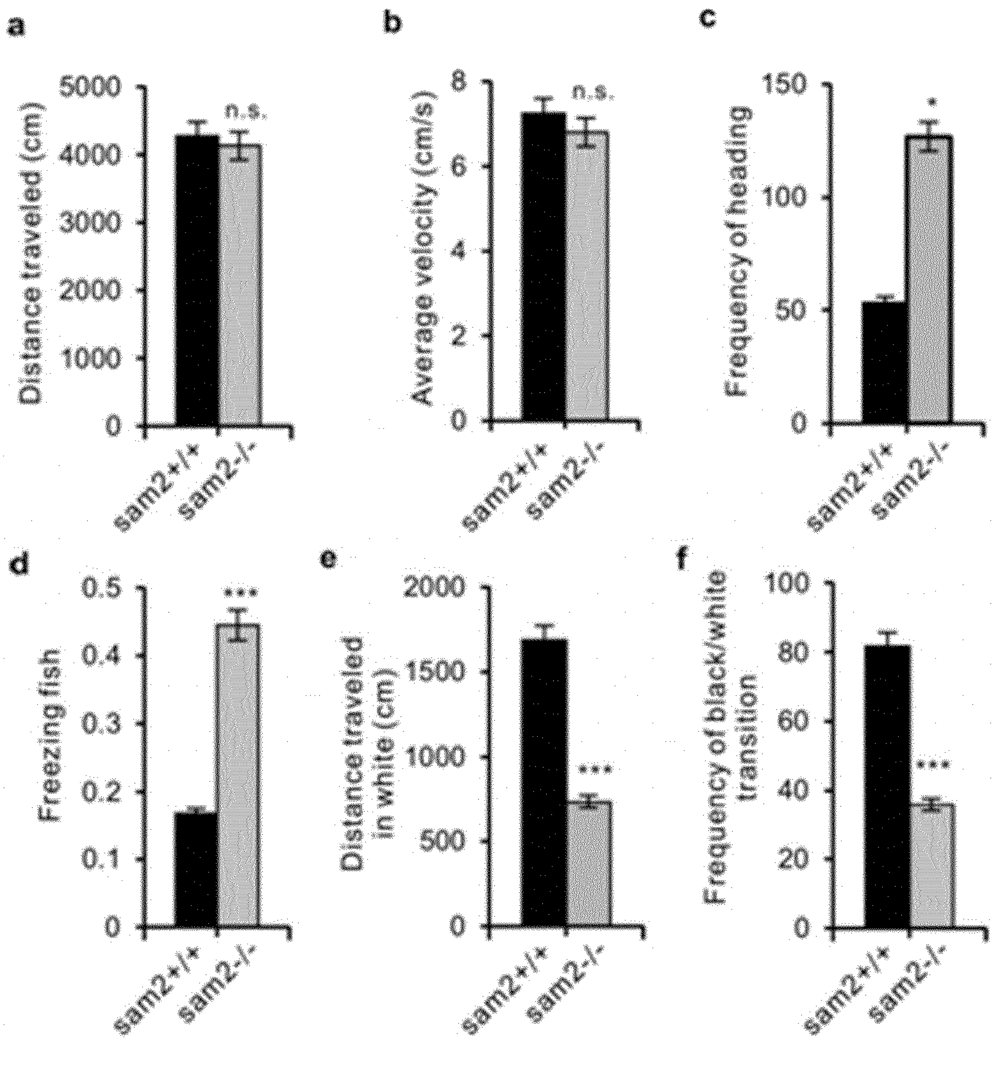
a
5000
4000
3000
2000
1000
0
Distance traveled (cm)
n.s.
sam2+/+
sam2-/-
b
8
6
4
2
0
Average velocity (cm/s)
n.s.
sam2+/+
sam2-/-
c
150
100
50
0
Frequency of heading
*
sam2+/+
sam2-/-
d
0.5
0.4
0.3
0.2
0.1
0
Freezing fish
***
sam2+/+
sam2-/-
e
2000
1500
1000
500
0
Distance traveled in white (cm)
***
sam2+/+
sam2-/-
f
100
80
60
40
20
0
Frequency of black/white transition
***
sam2+/+
sam2-/-

[Fig. 10]

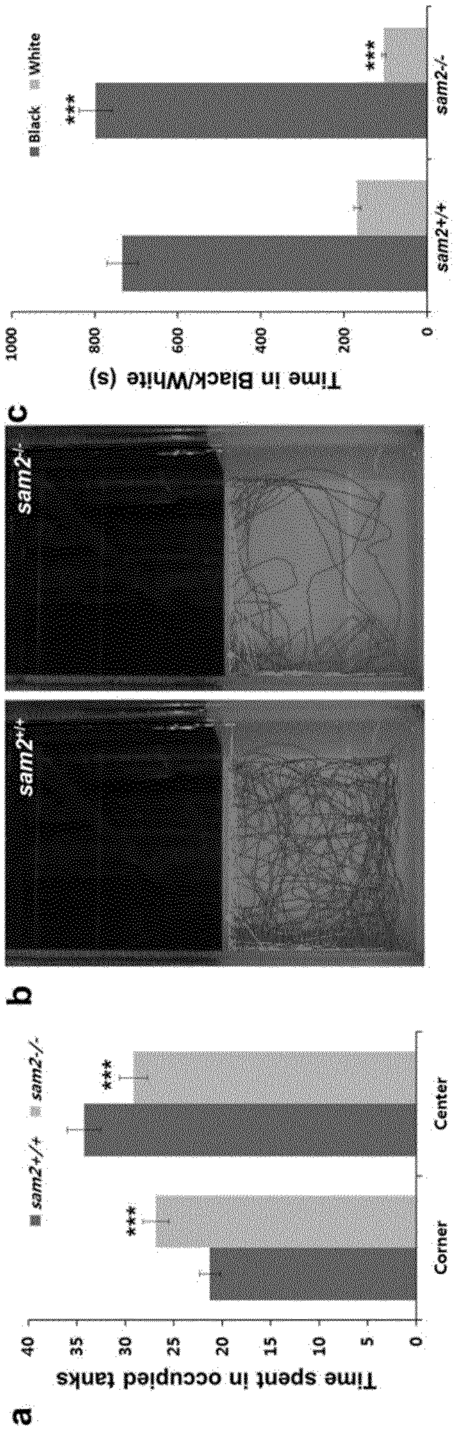
a
Time spent in occupied tanks
sam2+/+
sam2-/-
Corner
Center
***
b
sam2+/+
sam2-/-
c
Time in Black/White (s)
Black
White
sam2+/+
sam2-/-
***

[Fig. 11]

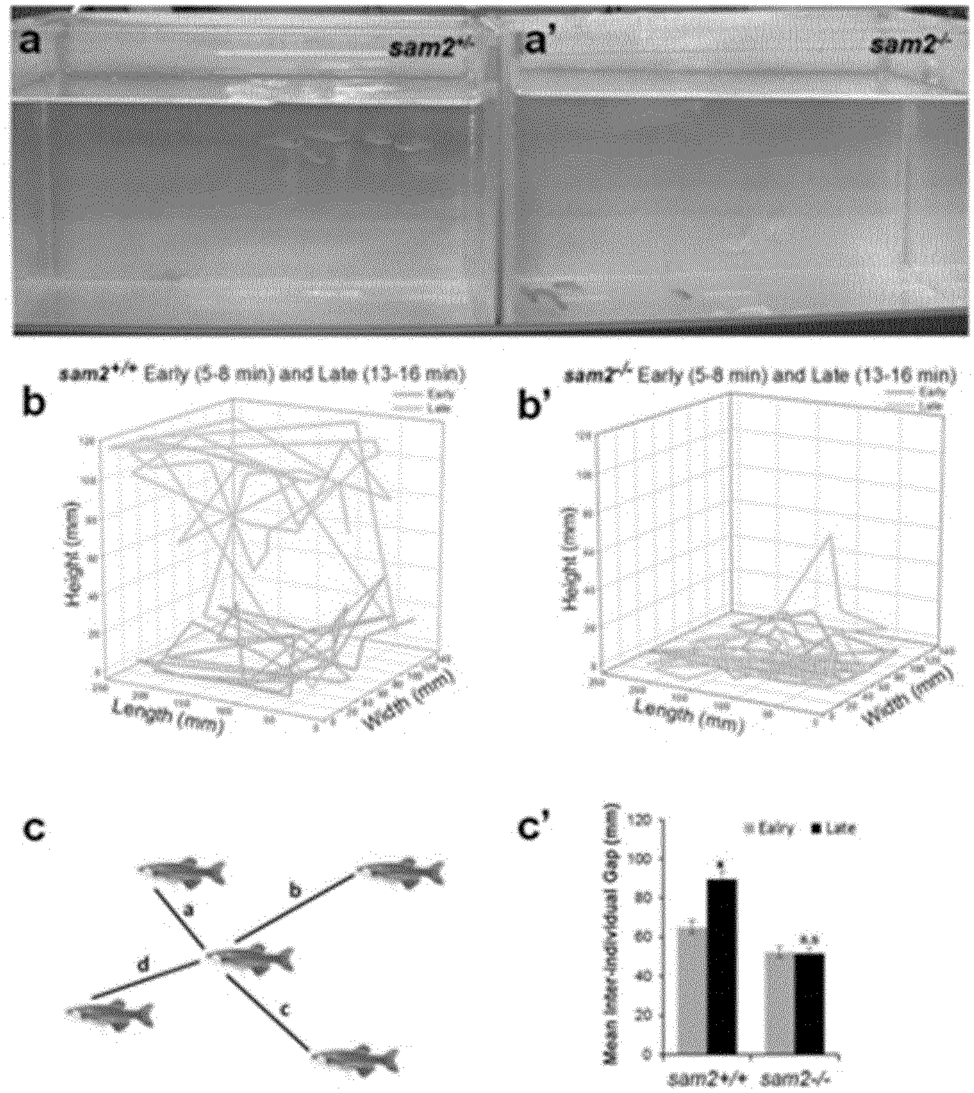
a
sam2+/-
a'
sam2-/-
b
sam2+/+ Early (5-8 min) and Late (13-16 min)
Height (mm)
Length (mm)
Width (mm)
b'
sam2-/- Early (5-8 min) and Late (13-16 min)
Height (mm)
Length (mm)
Width (mm)
c
a
b
c
d
c'
Mean Inter-individual Gap (mm)
Ealry
Late
120
100
80
60
40
20
0
sam2+/+
sam2-/-

[Fig. 12]

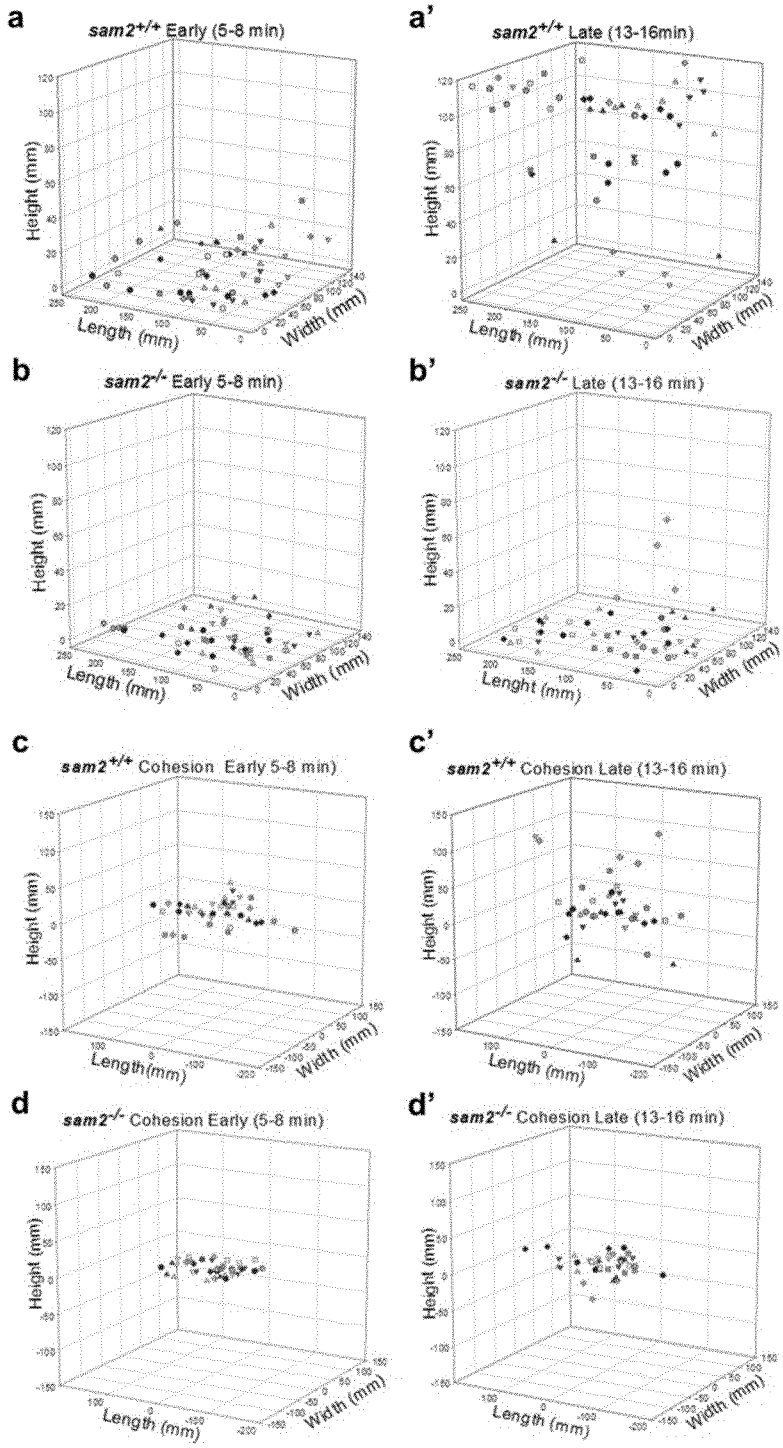
a
sam2+/+ Early (5-8 min)
a'
sam2+/+ Late (13-16min)
b
sam2-/- Early 5-8 min)
b'
sam2-/- Late (13-16 min)
c
sam2+/+ Cohesion Early 5-8 min)
c'
sam2+/+ Cohesion Late (13-16 min)
d
sam2-/- Cohesion Early (5-8 min)
d'
sam2-/- Cohesion Late (13-16 min)
Height (mm)
Length (mm)
Width (mm)

[Fig. 13]

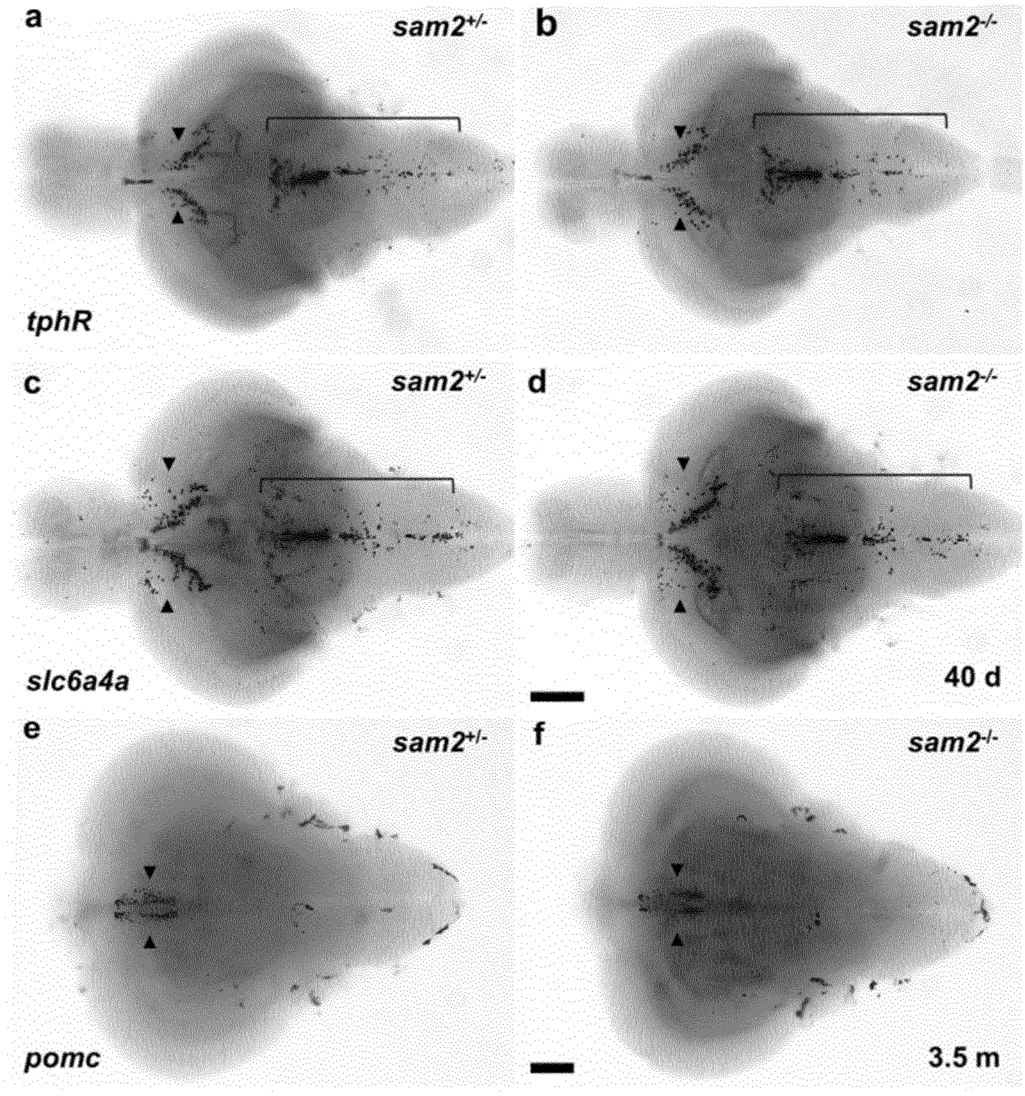
a
sam2+/-
b
sam2-/-
tphR
c
sam2+/-
d
sam2-/-
slc6a4a
40 d
e
sam2+/-
f
sam2-/-
pomc
3.5 m

[Fig. 14]

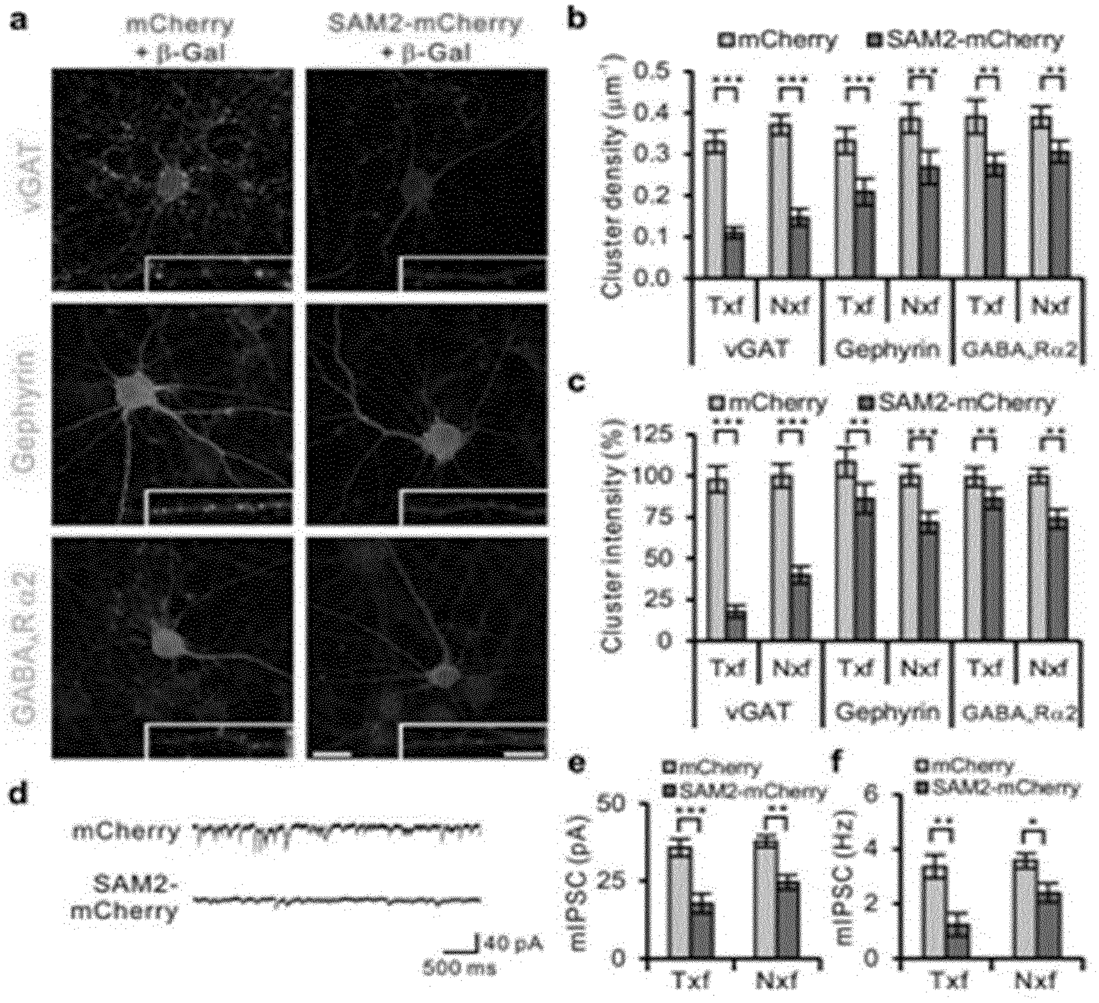
a
mCherry + β-Gal
SAM2-mCherry + β-Gal
vGAT
Gephyrin
GABA$_A$Rα2
b
mCherry
SAM2-mCherry
Cluster density (μm$^{-1}$)
Txf
Nxf
vGAT
Gephyrin
GABA$_A$Rα2
c
Cluster intensity (%)
d
mCherry
SAM2-mCherry
40 pA
500 ms
e
mIPSC (pA)
f
mIPSC (Hz)

[Fig. 15]

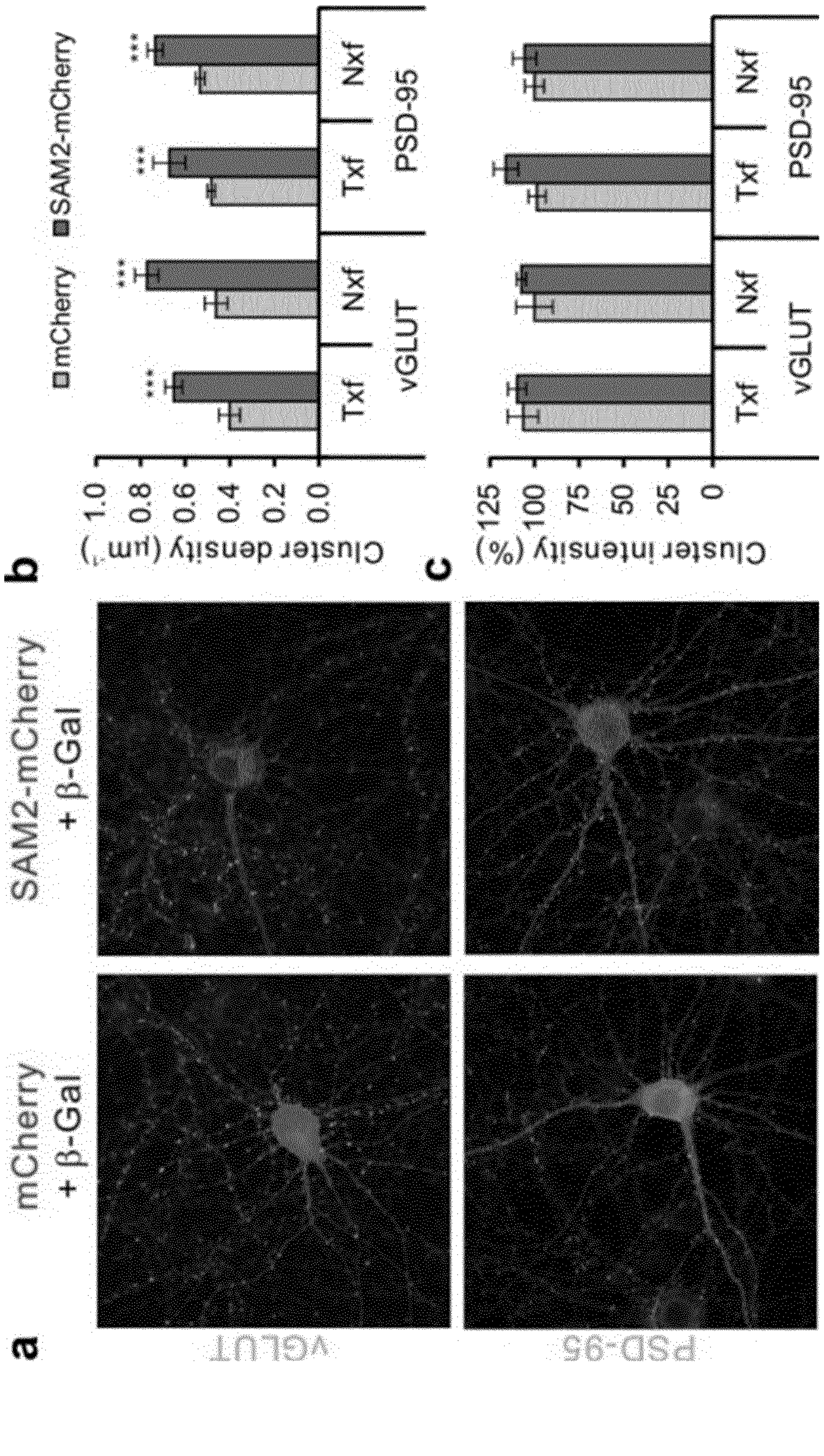
a
mCherry + β-Gal
SAM2-mCherry + β-Gal
vGLUT
PSD-95
b
mCherry
SAM2-mCherry
Cluster density (μm⁻¹)
1.0
0.8
0.6
0.4
0.2
0.0
Txf
Nxf
vGLUT
PSD-95
***
c
Cluster intensity (%)
125
100
75
50
25
0

[Fig. 16]

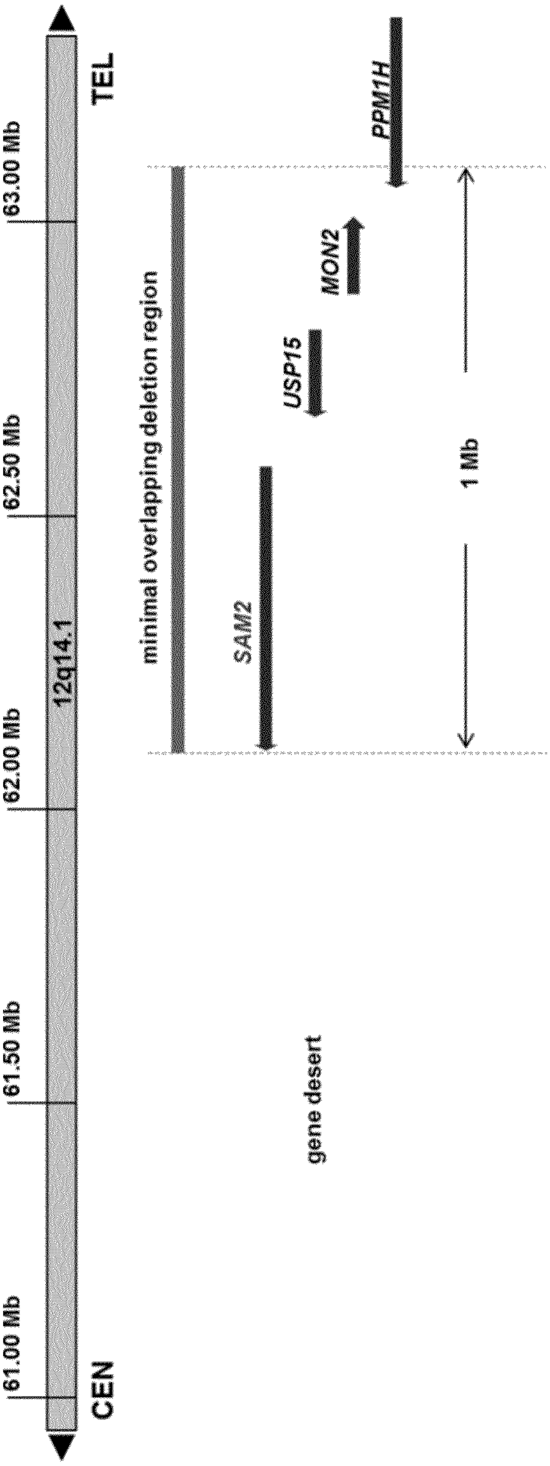
TEL
CEN
63.00 Mb
62.50 Mb
62.00 Mb
61.50 Mb
61.00 Mb
12q14.1
minimal overlapping deletion region
PPM1H
MON2
USP15
SAM2
1 Mb
gene desert

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20140031448


### Non-patent literature cited in the description


- **ROBERT. F.SCMIDT.** *Human physiology,* 366
- **MIN SEONGGIL.** *Latest Psychiatry,* 238-240
- *S. V., Pharmacol. Ther.,* 2000, vol. 88, 213-227
- **ROBERT. F. SCMIDT.** *Human physiology,* 366
- **HIKOSAKA.** *Nat. Rev. Neurosci,* 2010, vol. 11, 503-513
- **AMO, R. et al.** *J. Neurosci,* 2010, vol. 30, 1566-1574
- **BORG et al.** *Hum. Genet.,* 2005, vol. 118, 267-275
- **GERLAI. R.** *Behav Brain Res,* 2010, vol. 207, 223-231